# EUROPEAN PATENT SPECIFICATION

(11) **EP 4 498 936 B1**
(45) Date of publication and mention of the grant of the patent: **25.02.2026**
(21) Application number: 22718880.2
(22) Date of filing: 28.03.2022
(51) Int. Cl.: A61B 17/12

(54) **DELIVERY SYSTEMS FOR IMPLANTS**
FREISETZUNGSSYSTEME FÜR IMPLANTATE
SYSTÈMES D'ADMINISTRATION POUR IMPLANTS

(43) Date of publication of application: 05.02.2025
(73) Proprietor: Clearstream Technologies Limited, Enniscorthy, County Wexford (IE)
(72) Inventor: WHELAN, Michael, Enniscorthy, County Wexford (IE); O'CONNOR, Fearghal, Enniscorthy, County Wexford (IE); GILES, Ciaran, Enniscorthy, County Wexford (IE)
(74) Representative: Hoffmann Eitle
(86) International application number: PCT/EP2022/058090
(87) International publication number: WO 2023/186258

(56) References cited:
- EP-A1- 3 763 298
- GB-A- 2 533 087
- US-A1- 2010 106 178
- US-A1- 2016 302 794
- US-A1- 2017 245 864

## Description

### TECHNICAL FIELD

The present disclosure relates to delivery systems for implants. More particularly, the present disclosure relates to a delivery system for gripping and releasing an implant, and an embolization system actuatable to release an embolization device and having a flexible joint.

### BACKGROUND

When delivering medical implants to a location within a bodily lumen of a patient, the delivery system must often navigate tortuous anatomies, including sharp turns in the vasculature, for example at branches. Such tortuous anatomies cause strain to be experienced the delivery system caused by the bending forces exerted on the system as it navigates the anatomy. If the strain is transmitted to the medical implant or to the detach mechanism connecting the medical implant to the delivery system, this can cause premature detachment of the medical implant or even damage to the medical implant.

Furthermore, the delivery mechanism may comprise a detach mechanism which is actuatable to release the medical implant. The detach mechanism risks premature actuation, especially when strain is imparted onto the delivery system by bending forces experienced in the bodily lumen.

There is therefore a need for reducing the potential damage to medical implant delivery systems and medical implants during delivery in complex anatomies, and further a need for improving the reliability of detach mechanisms so that they are less likely to break or detach from the medical implant prematurely.

GB 2 533 087 A discloses a detachment mechanism designed to hold a coupling element of an implantable medical device until its desired release within a blood vessel. The detachment mechanism comprises a body portion having jaws which grip onto the coupling element in a coupling chamber and are biased into a closed position. An opening element, located in a separate chamber, is moveable from a first to a second position, opening the jaws by pulling the opening element against tapering internal surfaces of the body portion. The opening element may comprise a rounded head and may be attached to a trigger wire. The detachment mechanism may be formed from a cannula.

US 2016/302794 A1 discloses a delivery apparatus for delivering a medical device to the body of a patient. The apparatus has an outer catheter independently mobile from an inner sheath and an elongate member. The apparatus further has a gripper attached to the elongate member. The gripper may be manipulated by way of a handle to control the state of the medical device relative to the delivery apparatus. Through use of the handle, the apparatus may be moved from a connected state to a released state to deliver the medical device to a target site in the patient's body.

EP 3 763 298 A1 discloses clamp device for causing the hemostasis of a blood vessel.

US 2017/245864 A1 discloses a medical device comprising an expandable body for treating saccular vascular aneurysms and occluding segments of blood vessels and other biological conduits.

US 2010/106178 A1 discloses a device for occluding vascular vessels. The device is able to move from a first condition to a less compact, second condition in a vascular vessel so as to fully or partially prevent fluid from passing through the vessel.

### SUMMARY

According to a first aspect of the present disclosure, there is provided a delivery system for delivering and deploying an implant to a bodily lumen as defined in claim 1.

According to a second aspect of the present disclosure, there is provided an embolization system as defined in claim 10 comprising the delivery system according to the first aspect of the present disclosure and an embolization device.

### BRIEF DESCRIPTION OF THE DRAWINGS

To enable better understanding of the present disclosure, and to show how the same may be carried into effect, reference will now be made, by way of example only, to the accompanying schematic drawings, in which:
Fig. 1 shows a schematic view of an embolization system according to one or more embodiments shown and described herein;
Fig. 2A shows a schematic view of an embolization system in a bodily lumen according to one or more embodiments shown and described herein;
Fig. 2B shows the embolization system of Fig. 2A in another configuration;
Fig. 2C shows the embolization system of Fig. 2A in another configuration;
Fig. 3A shows a schematic view of another embolization system according to one or more embodiments shown and described herein;
Fig. 3B shows a schematic view of another embolization system according to one or more embodiments shown and described herein;
Fig. 3C shows a schematic view of another embolization system according to one or more embodiments shown and described herein;
Fig. 3D shows a schematic view of another embolization system according to one or more embodiments shown and described herein;
Fig. 4A shows a schematic view of another embolization system according to one or more embodiments shown and described herein;
Fig. 4B shows a schematic view of another embolization system according to one or more embodiments shown and described herein;
Fig. 4C shows a schematic view of another embolization system according to one or more embodiments shown and described herein;
Fig. 4D shows a schematic view of another embolization system according to one or more embodiments shown and described herein;
Fig. 5A shows a schematic view of another embolization system according to one or more embodiments shown and described herein;
Fig. 5B shows a schematic view of another embolization system according to one or more embodiments shown and described herein;
Fig. 5C shows a schematic view of another embolization system according to one or more embodiments shown and described herein;
Fig. 5D shows a schematic view of another embolization system according to one or more embodiments shown and described herein;
Fig. 6A shows a schematic view of another embolization system according to one or more embodiments shown and described herein;
Fig. 6B shows a schematic view of another embolization system according to one or more embodiments shown and described herein;
Fig. 6C shows a schematic view of another embolization system according to one or more embodiments shown and described herein;
Fig. 6D shows a schematic view of another embolization system according to one or more embodiments shown and described herein;
Fig. 7A shows a schematic view of another embolization system according to one or more embodiments shown and described herein;
Fig. 7B shows a schematic view of another embolization system according to one or more embodiments shown and described herein;
Fig. 7C shows a schematic view of another embolization system according to one or more embodiments shown and described herein;
Fig. 7D shows a schematic view of another embolization system according to one or more embodiments shown and described herein;
Fig. 8A shows a schematic view of another embolization system according to one or more embodiments shown and described herein;
Fig. 8B shows a schematic view of another embolization system according to one or more embodiments shown and described herein;
Fig. 8C shows a schematic view of another embolization system according to one or more embodiments shown and described herein;
Fig. 8D shows a schematic view of another embolization system according to one or more embodiments shown and described herein;
Fig. 9A shows a schematic view of another embolization system according to one or more embodiments shown and described herein;
Fig. 9B shows a schematic view of another embolization system according to one or more embodiments shown and described herein;
Fig. 9C shows a schematic view of another embolization system according to one or more embodiments shown and described herein;
Fig. 9D shows a schematic view of another embolization system according to one or more embodiments shown and described herein;
Fig. 10A shows a schematic view of a delivery system according to one or more embodiments shown and described herein but which is not covered by the invention of the appended claims.
Fig. 10B shows the delivery system of Fig. 10A in a second configuration;
Fig. 11A shows a schematic view of a delivery system in a bodily lumen according to one or more embodiments shown and described herein but which is not covered by the invention of the appended claims.
Fig. 11B shows the delivery system of Fig. 10A in a second configuration;
Fig. 11C shows the delivery system of Fig. 10A in a third configuration;
Fig. 12A shows a schematic view of a delivery system according to one or more embodiments shown and described herein;
Fig. 12B shows the delivery system of Fig. 12A in a second configuration;
Fig. 13A shows a schematic view of a delivery system according to one or more embodiments shown and described herein;
Fig. 13B shows the delivery system of Fig. 13A in a second configuration;
Fig. 14A shows a schematic view of a delivery system according to one or more embodiments shown and described herein;
Fig. 14B shows the delivery system of Fig. 14A in a second configuration;
Fig. 15A shows a schematic view of a delivery system according to one or more embodiments shown and described herein but which is not covered by the invention of the appended claims.
Fig. 15B shows the delivery system of Fig. 15A in a second configuration;
Fig. 16A shows a schematic view of a delivery system according to one or more embodiments shown and described herein but which is not covered by the invention of the appended claims.
Fig. 16B shows the delivery system of Fig. 16A in a second configuration;
Fig. 17A shows a schematic view of a delivery system according to one or more embodiments shown and described herein but which is not covered by the invention of the appended claims.
Fig. 17B shows the delivery system of Fig. 17A in a second configuration;
Fig. 18 shows a schematic view of an embolization device according to one or more embodiments shown and described herein;
Fig. 19A shows a schematic view of an embolization system according to one or more embodiments shown and described herein;
Fig. 19B shows a schematic view of an embolization system according to one or more embodiments shown and described herein;
Fig. 20A shows a schematic diagram of a breakable detachment element for an embolization system according to one or more embodiments shown and described herein;
Fig. 20B shows a schematic diagram of another breakable detachment element for an embolization system according to one or more embodiments shown and described herein; and
Fig. 20C shows a schematic diagram of another breakable detachment element for an embolization system according to one or more embodiments shown and described herein.

### DETAILED DESCRIPTION

As disclosed herein, the term "skeleton" may be understood to mean a structure which is configured to provide structural support to a layer of material formed on the skeleton. The skeleton may comprise a plurality of struts providing the structural support.

As disclosed herein, the term "flow-restricting layer" may be understood as a component part of an embolization device which is configured to extend across a substantial cross-section of the embolization device when deployed in a bodily lumen such that blood-flow through the lumen is at least partially restricted by the component.

As disclosed herein, the term "delivery element" may be understood as any element configured to fit inside a delivery catheter which is able to push a medical implant through the delivery catheter to a distal end of the delivery catheter.

As disclosed herein, the term "breakable detachment element" may be understood as an element configured to break upon application of a predetermined force. More specifically, it may refer to any element which is configured to break irreversibly such that two elements which are connected by the detachment element are separated irreversibly. The breakable detachment element may connect two elements of a system such that when a force (for example a shear force or twisting force) is applied to the system, the breakable detachment element preferentially breaks before the other elements of the system.

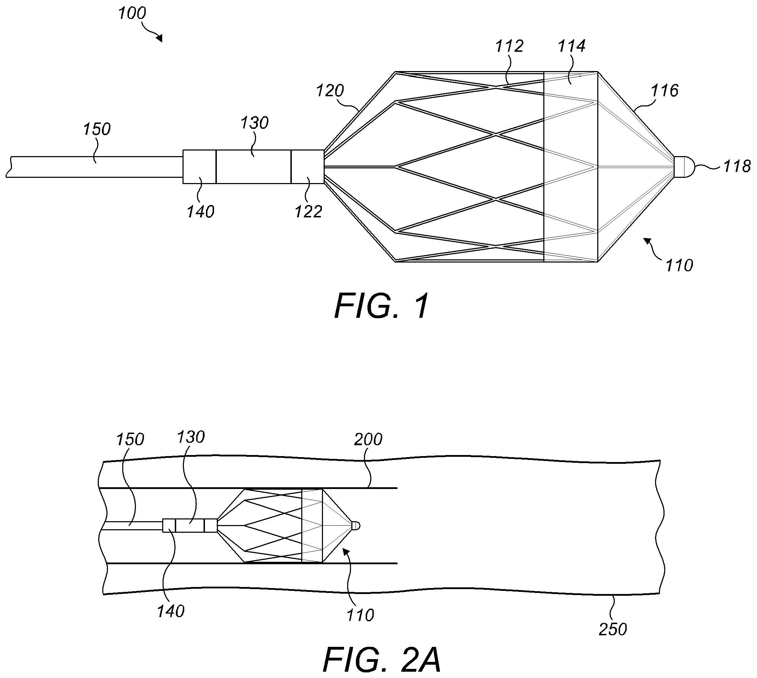

Fig. 1 shows an embolization system 100 comprising an embolization device 110 and a detach mechanism 140 for connecting the embolization device 110 to a delivery element 150 (which may be, for example, a wire, ribbon or similar configured to extend through a delivery catheter). The embolization system 100 may further comprise a flexible joint 130 having a higher flexibility than the embolization device 110, configured to allow the embolization device 110 to tilt with respect to the delivery element 150. The embolization device 110 may be considered a "Micro Vascular Plug System" (MVP) suitable for peripheral embolization, such as the MVP manufactured by Medtronic.

Whilst the flexible joint 130 is shown in Fig. 1 as being distal of the detach mechanism 140, in other embodiments the detach mechanism 140 may be distal of the flexible joint 130.

The embolization device 110 may further comprise a self-expandable skeleton 112 (which may comprise, for example, a plurality of interconnected or braided struts forming a self-expanding cage). The embolization device 110 may further comprise a flow-restricting layer 114 mounted onto the skeleton 112. The embolization device 110 has a collapsed delivery configuration in which the embolization device 110 is configured to fit inside a delivery catheter, and an expanded deployed configuration in which the skeleton 112 is configured to anchor the embolization device 110 to a bodily lumen. The flow restricting layer 114, being mounted to the skeleton 112, is moved between the collapsed delivery configuration and the expanded deployed configuration by the self-expandable skeleton 112. In the expanded deployed configuration, the flow restricting layer 114 extends at least partially and across the bodily lumen to restrict blood flow through the bodily lumen. In some embodiments, the flow restricting layer 114 is configured to extend wholly across the bodily lumen in the expanded deployed configuration.

The self-expandable skeleton 112 may comprise an anchoring portion which is configured to contact the bodily lumen in the expanded deployed configuration and provide the anchoring force of the embolization device 110. The anchoring portion may be, for example, a cylindrical shape in the expanded deployed configuration. The self-expandable skeleton 112 may also comprise one or more of a proximal tapered portion 120 and a distal tapered portion 116. The distal tapered portion 116 may terminate at a tip 118. The tip 118 may be an atraumatic shape, such as a rounded shape, and/or may be formed of a material which is softer than the material of the skeleton 112, to reduce the risk of perforation of vessel walls as the embolization device 110 is deployed. The proximal tapered portion 120 improves retrievability of the embolization device 110 by allowing it to be re-collapsed by a delivery catheter. The proximal tapered portion 120 may terminate at a proximal fixing element 122 which connects the proximal end of the skeleton 112 to the flexible joint 130 or the detach mechanism 140. Alternatively, the proximal end of the skeleton 112 may be directly connected to the flexible joint 130 or detach mechanism 140. In some embodiments, instead of one or both of the tapered portions the embolization device 110 comprises a portion that extends radially inwards of the anchoring portion (i.e. does not have longitudinal extent) to connect the anchoring portion to the proximal fixing element 122, flexible joint 130 or detach mechanism 140. The self-expandable skeleton 112 may be made of any self-expandable material, for example, stainless steel or nitinol.

The embolization device 110 may comprise one or more radiopaque markers to assist locating the embolization device 110 when deployed inside the bodily lumen. For example, the proximal fixing element 122 and/or the distal tip 118 and/or the skeleton 112 may be made of a radiopaque material.

The detach mechanism 140 is configured to connect the embolization device 110 to the delivery element 150 and is actuatable to release the embolization device 110 from the delivery element 150. The detach mechanism 140 may be a reversible detach mechanism such as a screw thread (i.e. the detachment may be reversable), or the detach mechanism 140 may be an irreversible detach mechanism such as an electrolytic element, as disclosed in further detail herein. Any of the embolization systems disclosed herein may comprise any such detach mechanism.

The flexible joint 130 may be provided as part of the embolization device 110 or may be provided as part of the delivery element 150.

The flow restricting layer 114 is illustrated as covering the skeleton 112 from a distal end of the embolization device 110 to a point along the anchoring portion of the skeleton 112. In other embodiments, the flow restricting layer 114 may extend from the proximal end of the embolization device to a point along the anchoring portion of the skeleton 112, or may cover the entire length of skeleton 112. The flow restricting layer 114 may comprise at least one longitudinal end which is closed such that in the expanded deployed configuration, the flow restricting layer 114 extends across the entire diameter of the bodily lumen. The flow restricting layer 114 may be any suitable flexible layer which is able to move between the collapsed delivery configuration and the expanded deployed configuration, and may for example be a flexible polymer, and more specifically PTFE or polyurethane, polyethylene or a composite thereof. The layer 114 may be separately formed and mounted to the skeleton 112 by welding, adhesive, tying or any other suitable means for mounting to the skeleton 112, on the inner side or the outer side of the skeleton 112. Alternatively, the layer 114 may be formed on the skeleton by dip-casting.

Fig. 2A shows an embolization system 100 (for example the embolization system 100 described with respect to Fig. 1) in a collapsed delivery configuration inside a delivery catheter 200. In use, the delivery catheter 200 is advanced through the vasculature of a patient to a target site. The embolization device 110 is then collapsed into the collapsed delivery configuration and advanced through to the distal tip of the delivery catheter 200 by delivery element 150.

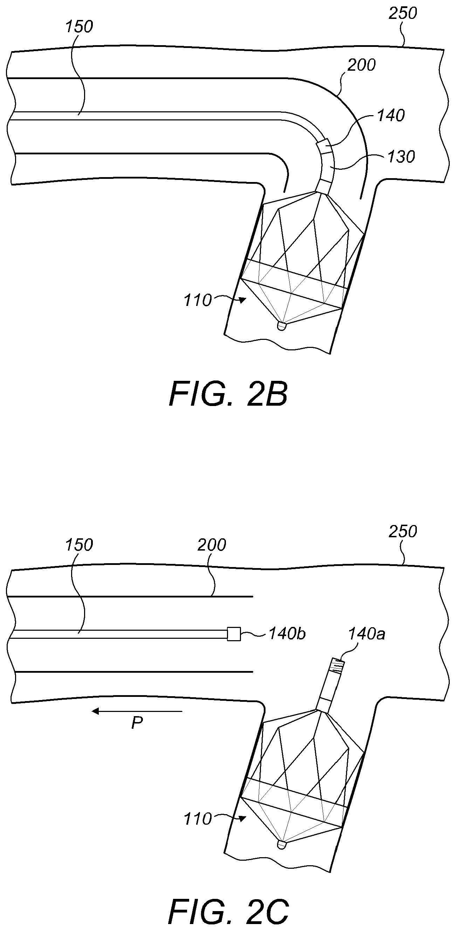

Fig. 2B shows the embolization system 100 in a partially deployed configuration in which the embolization device 110 has been deployed from the distal tip of the delivery catheter 200 and has expanded to the expanded deployed configuration in which the embolization device 110 is anchored to the vessel wall 250 of the bodily lumen at the target site. As in the illustrated embodiment, in some cases the deployment site of the embolization device 110 is tilted at a large angle compared to the delivery element 150. Such a large tilt angle can exert large bending forces on the embolization device 110 and may cause damage to the embolization device 110 or cause it to detach from delivery element 150 prematurely. In the illustrated embodiment, as the embolization system 100 is provided with flexible joint 130 which has a higher flexibility than embolization device 110, the bending is taken up primarily by the flexible joint 130, reducing or avoiding any bending forces being transmitted to the embolization device 110 and thereby avoiding damage to the embolization device 110 upon deployment at a substantial tilt angle, or premature detachment of the embolization device 110.

Once it is determined that the embolization device 110 is correctly located in the bodily lumen, the detach mechanism 140 can be actuated by the user to detach the embolization device 110 from the delivery element 150, as shown in Fig. 2C. The delivery element 150 and the delivery catheter 200 are retracted in a proximal direction P and removed from the bodily lumen, and the embolization device 110 is anchored to the bodily lumen in the desired location.

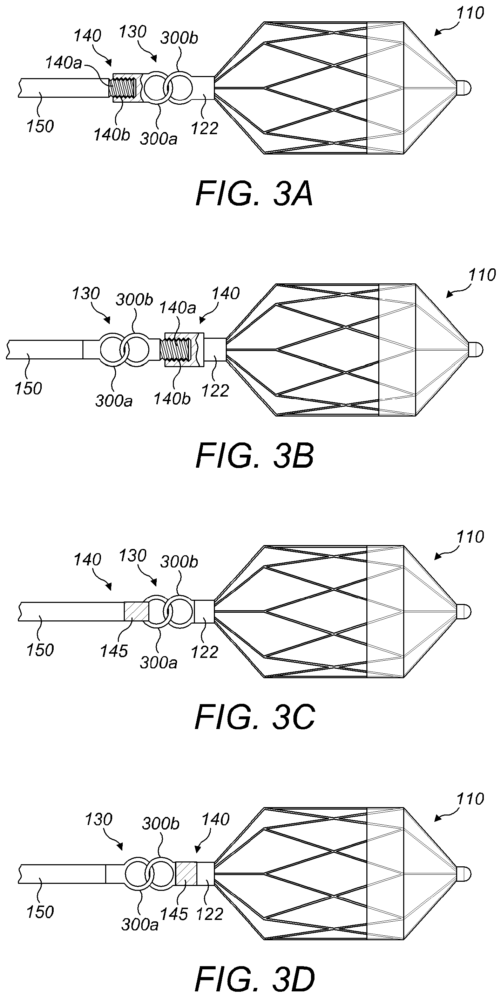

Fig. 3A shows an embodiment of the embolization system 100 described with respect to Fig. 1. In the embodiment of Fig. 3A, the flexible joint 130 is provided distally to the detach mechanism 140. In the illustrated embodiment, the flexible joint 130 may comprise an articulating joint, which comprises interlocking loops 300a and 300b. Loop 300b is connected to the embolization device 110 and loop 300a is connected to the detach mechanism 140. The interlocking loops 300a, 300b may be made of any suitable material such as a polymer or metal. Detach mechanism 140 may include a female screw thread 140b which is configured to connect to a male screw thread 140a on a delivery element 150 (in other embodiments the detach mechanism 140 comprises a male screw thread connected to the flexible joint 130, configured to connect to a female screw thread on the delivery element 150). The loop 300b may be formed on the proximal fixing element 122 or may be formed directly on the skeleton 114 of embolization device 110.

The deployment method disclosed with respect to Figs. 2A to 2C can be used for the embolization system 100 shown in Fig. 3A. The interlocking loops 300a and 300b allow the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 3B shows another embodiment of the embolization system 100 described with respect to Fig. 1. In the embodiment of Fig. 3B, the flexible joint 130 is provided proximally to the detach mechanism 140. In the depicted embodiment, the flexible joint 130 may comprise an articulating joint, which comprises interlocking loops 300a and 300b. The interlocking loops 300a, 300b may be made of any suitable material such as a polymer or metal. Loop 300b, in the present embodiment, is connected to the detach mechanism 140 and loop 300a is connected to the delivery element 150. Detach mechanism 140 may include a female screw thread 140b connected to embolization device 110 which is configured to connect to a male screw thread 140a connected to loop 300b (in other embodiments the detach mechanism 140 comprises a male screw thread connected to the embolization device 110, configured to connect to a female screw thread connected to the loop 300b). the female screw thread 140b may be formed on the proximal fixing element 122 or may be formed directly on the skeleton 114 of embolization device 110.

The deployment method disclosed with respect to Figs. 2A to 2C can be used for the embolization system 100 shown in Fig. 3B. The interlocking loops 300a and 300b allow the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 3C shows another embodiment of the embolization system described with respect to Fig. 1. In the embodiment of Fig. 3C, the flexible joint 130 is provided distally to the detach mechanism 140. The flexible joint 130 may comprise an articulating joint, which comprises interlocking loops 300a and 300b. The interlocking loops 300a, 300b may be made of any suitable material such as a polymer or metal. Loop 300b is connected to the embolization device 110 and loop 300a is connected to the detach mechanism 140. The loop 300b may be formed on the proximal fixing element 122 or may be formed directly on the skeleton 114 of embolization device 110. Detach mechanism 140 may comprise an electrolytic element 145 connecting the flexible joint 130 and the delivery element 150. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110. It is noted that in some embodiments, loop 300a or 300b may be formed of the electrolytic material 145, thus acting as both the flexible joint 130 and the detach mechanism 140.

The deployment method disclosed with respect to Figs. 2A to 2C can be used for the embolization system 100 shown in Fig. 3C. The interlocking loops 300a and 300b allow the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

Fig. 3D shows another embodiment of the embolization system described with respect to Fig. 1. In the embodiment of Fig. 3D, the flexible joint 130 is provided proximally to the detach mechanism 140. The flexible joint 130 may comprise an articulating joint, which comprises interlocking loops 300a and 300b. The interlocking loops 300a, 300b may be made of any suitable material such as a polymer or metal. Loop 300b is connected to the detach mechanism 140 and loop 300a is connected to the delivery element 150. Detach mechanism 140 comprises an electrolytic element 145 connecting the flexible joint 130 and the embolization device 110. Loop 300b may be formed on the electrolytic element 145 or one or more of loops 300a, 300b may themselves be made of electrolytic material such that it acts as both the joint 130 and the electrolytic element 145. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The loops 300a, 300b may be electrically conductive themselves or a flexible conductive wire may extend across the joint 130 to electrically connect the electrolytic element 145. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110.

The deployment method disclosed with respect to Figs. 2A to 2C can be used for the embolization system 100 shown in Fig. 3D. The interlocking loops 300a and 300b allow the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

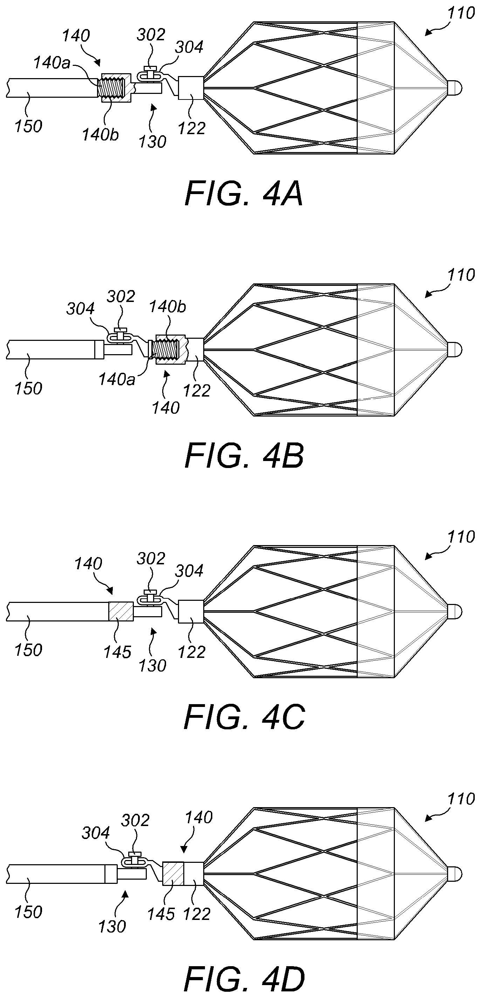

Fig. 4A shows an embodiment of the embolization system 100 described with respect to Fig. 1. In the embodiment of Fig. 4A, the flexible joint 130 is provided distally to the detach mechanism 140. The flexible joint 130 may comprise an articulating joint, which comprises a hinge pin 302 and a hinge loop 304 connected to the hinge pin 302. The hinge pin 302 and hinge loop 304 may be made of any suitable material such as a polymer or metal. Hinge loop 304 is connected to the embolization device 110 (e.g. formed on proximal fixing element 122 or directly on skeleton 114). Detach mechanism 140 may comprise a female screw thread 140b which is configured to connect to a male screw thread 140a on a delivery element 150 (in other embodiments the detach mechanism 140 comprises a male screw thread connected to the flexible joint 130, configured to connect to a female screw thread on the delivery element 150). Hinge loop 304 may be formed on the proximal fixing element 122 or may be formed directly on the skeleton 114 of embolization device 110. In some embodiments, the hinge pin 302 is formed on the proximal fixing element 122 or directly on the skeleton 114, and the hinge loop 304 is connected to the detach mechanism 140.

The deployment method disclosed with respect to Figs. 2A to 2C can be used for the embolization system 100 shown in Fig. 4A. The hinge pin 302 and hinge loop 304 allow the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 4B shows an embodiment of the embolization system 100 described with respect to Fig. 1. In the embodiment of Fig. 4B, the flexible joint 130 is provided proximally to the detach mechanism 140. The flexible joint may comprise an articulating joint, which comprises a hinge pin 302 and a hinge loop 304 connected to the hinge pin 302. The hinge pin 302 and hinge loop 304 may be made of any suitable material such as a polymer or metal. Hinge loop 304 is connected to detach mechanism 140 and hinge pin 302 is connected to the delivery element 150. Detach mechanism 140 may comprise a female screw thread 140b formed on the proximal fixing element 122 or directly on the skeleton 114, which is configured to connect to a male screw thread 140a connected to flexible joint 130 (in other embodiments the detach mechanism 140 comprises a male screw thread connected to the embolization device 110, configured to connect to a female screw thread connected to the delivery element 150 via flexible joint 130). In some embodiments, the hinge pin 302 is connected to detach mechanism 140, and the hinge loop 304 is connected to the delivery element 150.

The deployment method disclosed with respect to Figs. 2A to 2C can be used for the embolization system 100 shown in Fig. 4B. The interlocking hinge pin 302 and hinge loop 304 allow the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 4C shows an embodiment of the embolization system 100 described with respect to Fig. 1. In the embodiment of Fig. 4C, the flexible joint 130 is provided distally to the detach mechanism 140. The flexible joint 130 may comprise an articulating joint, which comprises a hinge pin 302 and a hinge loop 304 connected to the hinge pin 302 The hinge pin 302 and hinge loop 304 may be made of any suitable material such as a polymer or metal. Hinge loop 304 is connected to embolization device 110 (either via proximal fixing element 122 or directly to the skeleton 114). Hinge pin 302 is connected to detach mechanism 140. In other embodiments hinge loop 304 is connected to detach mechanism 140 and hinge pin 302 is connected to embolization device 110. Detach mechanism 140 may include an electrolytic element 145 connecting the flexible joint 130 and the delivery element 150. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110. It is noted that in some embodiments, the more proximal of the hinge pin 302 or hinge loop 304 may be formed of the electrolytic material 145, the flexible joint 130 thus acting as both the flexible joint 130 and the detach mechanism 140.

The deployment method disclosed with respect to Figs. 2A to 2C can be used for the embolization system 100 shown in Fig. 4C. The interlocking hinge pin 302 and hinge loop 304 allow the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

Fig. 4D shows another embodiment of the embolization system described with respect to Fig. 1. In the embodiment of Fig. 4D, the flexible joint 130 is provided proximally to the detach mechanism 140. The flexible joint 130 may comprise an articulating joint, which comprises a hinge pin 302 and a hinge loop 304 connected to the hinge pin 302. The hinge pin 302 and hinge loop 304 may be made of any suitable material such as a polymer or metal. Hinge loop 304 is connected to detach mechanism 140 and hinge pin 302 is connected to delivery element 150. In other embodiments, hinge loop 304 is connected to delivery element 150 and hinge pin 302 is connected to detach mechanism 140. Detach mechanism 140 comprises an electrolytic element 145 connecting the flexible joint 130 and the embolization device 110. The more distal of the hinge pin 302 and hinge loop 304 may be formed on the electrolytic element 145 or may itself be made of electrolytic material such that it acts as both the joint 130 and the electrolytic element 145. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The hinge pin 302 and hinge loop 304 may be electrically conductive themselves or a flexible conductive wire may extend across the joint 130 to electrically connect the electrolytic element 145. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110.

The deployment method disclosed with respect to Figs. 2A to 2C can be used for the embolization system 100 shown in Fig. 4D. The interlocking hinge pin 302 and hinge loop 304 allow the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

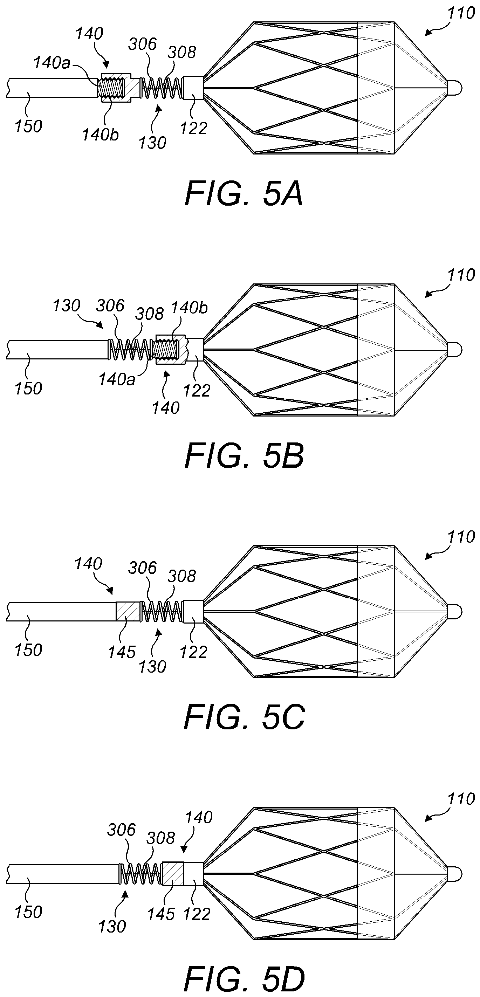

Fig. 5A shows an embodiment of the embolization system 100 described with respect to Fig. 1. In the embodiment of Fig. 5A, the flexible joint 130 is provided distally to the detach mechanism 140. The flexible joint 130 in the present embodiment comprises a resiliently deformable joint, and more particularly a spring 306 (although any suitable resiliently deformable element may be used such as an elastic material). The spring 306 may be made of any suitable material such as a polymer or metal. Optionally, the flexible joint 130 may additionally comprise inextensible wire or thread 308 (made of e.g. a polymer or metal) connecting the detach mechanism 140 and the embolization device 110, which inhibits the spring 306 from being overstretched. The spring 306 is connected to the detach mechanism 140 at a proximal end and embolization device 110 via the proximal fixing element 122 or skeleton 112 at a distal end. Detach mechanism 140 may comprise a female screw thread 140b which is configured to connect to a male screw thread 140a on a delivery element 150 (in other embodiments the detach mechanism 140 comprises a male screw thread connected to the flexible joint 130, configured to connect to a female screw thread on the delivery element 150).

The deployment method disclosed with respect to Figs. 2A to 2C can be used for the embolization system 100 shown in Fig. 5A. Spring 306 allows the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C (whilst the flexible spring 306 allows the embolization device 110 to rotate axially relative to the delivery element 150, with sufficient rotation of the delivery element 150 tension in the spring 306 becomes sufficient to allow the detach mechanism 140 to unscrew with further rotation).

Fig. 5B shows an embodiment of the embolization system 100 described with respect to Fig. 1. In the embodiment of Fig. 5B, the flexible joint is provided proximally to the detach mechanism 140. The flexible joint may comprise a resiliently deformable joint, and more particularly a spring 306 (although any suitable resiliently deformable element may be used such as an elastic material). The spring 306 may be made of any suitable material such as a polymer or metal. Optionally, the flexible joint 130 may additionally comprise inextensible wire or thread 308 (made of e.g. a polymer or metal) connecting the detach mechanism 140 and the embolization device 110, which inhibits the spring 306 from being overstretched. The spring 306 is connected to the detach mechanism at a distal end and the delivery element at a proximal end. Detach mechanism 140 may comprise a female screw thread 140b formed on the proximal fixing element 122 or directly on the skeleton 114, which is configured to connect to a male screw thread 140a connected to flexible joint 130 (in other embodiments the detach mechanism 140 comprises a male screw thread connected to the embolization device 110, configured to connect to a female screw thread connected to the delivery element 150 via flexible joint 130).

The deployment method disclosed with respect to Figs. 2A to 2C can be used for the embolization system 100 shown in Fig. 5B. The resiliently deformable element allows the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 5C shows an embodiment of the embolization system 100 described with respect to Fig. 1. In the embodiment of Fig. 5C, the flexible joint is provided distally to the detach mechanism. The flexible joint may comprise a resiliently deformable joint, and more particularly a spring 306 (although any suitable resiliently deformable element may be used such as an elastic material). The spring 306 may be made of any suitable material such as a polymer or metal. Optionally, the flexible joint 130 may additionally comprise inextensible wire or thread 308 (made of e.g. a polymer or metal) connecting the detach mechanism 140 and the embolization device 110, which inhibits the spring 306 from being overstretched. The spring 306 is connected to the embolization device 110 at a distal end (e.g. via proximal fixing element 122 or skeleton 114) and the detach mechanism 140 at a proximal end. Detach mechanism 140 may comprise an electrolytic element 145 connecting the flexible joint 130 and the delivery element 150. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110. It is noted that in some embodiments, the spring 306 (and the wire or thread 308) may be made of the electrolytic material 145, the flexible joint 130 thus acting as both the flexible joint 130 and the detach mechanism 140.

The deployment method disclosed with respect to Figs. 2A to 2C can be used for the embolization system 100 shown in Fig. 5C. The resiliently deformable element allows the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

Fig. 5D shows another embodiment of the embolization system described with respect to Fig. 1. In the embodiment of Fig. 5D, the flexible joint 130 is provided proximally to the detach mechanism 140. The flexible joint 130 may comprise a resiliently deformable joint, and more particularly a spring 306 (although any suitable resiliently deformable element may be used such as an elastic material). The spring 306 may be made of any suitable material such as a polymer or metal. Optionally, the flexible joint 130 may additionally comprise inextensible wire or thread 308 (made of e.g. a polymer or metal) connecting the detach mechanism 140 and the embolization device 110, which inhibits the spring 306 from being overstretched. The spring 306 is connected to the detach mechanism 140 at a distal end and the delivery element 150 at a proximal end. Detach mechanism 140 may comprise an electrolytic element 145 connecting the flexible joint 130 and the embolization device 110. The spring 306 and wire or thread 308 (where present) may be formed on the electrolytic element 145 or may themselves be made of electrolytic material such that they act as both the joint 130 and the electrolytic element 145. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The spring 306 and/or the thread 308 may be electrically conductive themselves or a flexible conductive wire may extend across the joint 130 to electrically connect the electrolytic element 145. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110.

The deployment method disclosed with respect to Figs. 2A to 2C can be used for the embolization system 100 shown in Fig. 5D. The resiliently deformable element allows the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

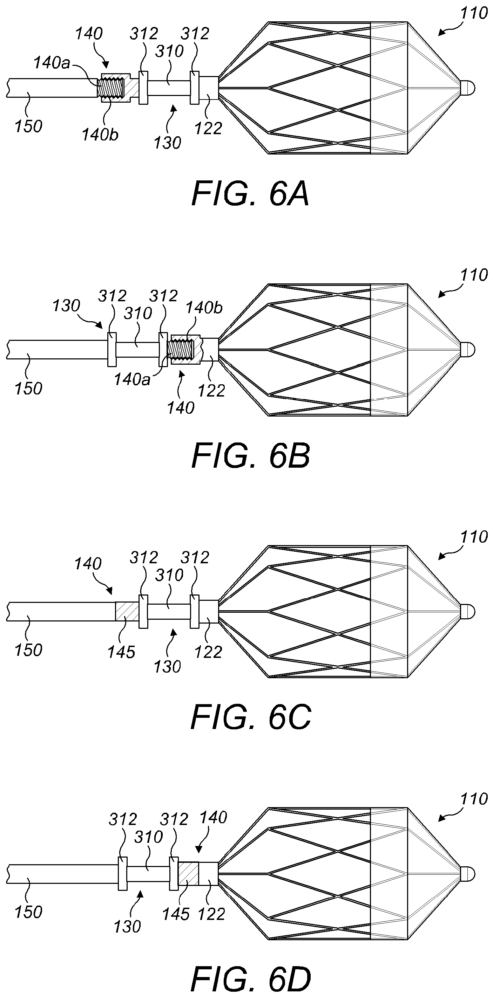

Fig. 6A shows an embodiment of the embolization system 100 described with respect to Fig. 1. In the embodiment of Fig. 6A, the flexible joint 130 is provided distally to the detach mechanism 140. The flexible joint 130 may comprise a flexible material 310 (for example a wire, thread or ribbon). The flexible material 310 may be made of any suitable material such as a polymer or metal. The flexible material 310 is connected to the detach mechanism 140 at a proximal end and the embolization device 110 (proximal fixing element 122 or skeleton 112) at a distal end. The flexible material 310 may be crimped to the detach mechanism 140 and the embolization device by crimped hypotubes 312. Alternatively, the elongate material 310 may be attached by adhesive or welding. Detach mechanism 140 comprises a female screw thread 140b which is configured to connect to a male screw thread 140a on a delivery element 150 (in other embodiments the detach mechanism 140 comprises a male screw thread connected to the flexible joint 130, configured to connect to a female screw thread on the delivery element 150).

The deployment method disclosed with respect to Figs. 2A to 2C can be used for the embolization system 100 shown in Fig. 6A. Flexible elongate material 310 allows the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 6B shows an embodiment of the embolization system 100 described with respect to Fig. 1. In the embodiment of Fig. 6B, the flexible joint 130 is provided proximally to the detach mechanism 140. The flexible joint may comprise a flexible material 310 (for example a wire, thread or ribbon). The flexible material 310 may be made of any suitable material such as a polymer or metal. The flexible material 310 is connected to the detach mechanism 140 at a distal end and the elongate element 150 at a proximal end. The flexible material 310 may be crimped to the detach mechanism 140 and the delivery element 150 by crimped hypotubes 312. Alternatively, the flexible material 310 may be attached by adhesive or welding. Detach mechanism 140 comprises a female screw thread 140b formed on the proximal fixing element 122 or directly on the skeleton 114, which is configured to connect to a male screw thread 140a connected to flexible joint 130 (in other embodiments the detach mechanism 140 comprises a male screw thread connected to the embolization device 110, configured to connect to a female screw thread connected to the delivery element 150 via flexible joint 130).

The deployment method disclosed with respect to Figs. 2A to 2C can be used for the embolization system 100 shown in Fig. 6B. The flexible material 310 allows the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 6C shows an embodiment of the embolization system 100 described with respect to Fig. 1. In the embodiment of Fig. 6C, the flexible joint 130 is provided distally to the detach mechanism 140. The flexible joint 130 may comprise a flexible material 310 (for example a wire, thread or ribbon). The flexible material 310 may be made of any suitable material such as a polymer or metal. The flexible material 310 is connected to the detach mechanism 140 at a proximal end and the embolization device 110 (proximal fixing element 122 or skeleton 112) at a distal end. The flexible material 310 may be crimped to the detach mechanism 140 and the embolization device by crimped hypotubes 312 or by adhesive or welding. Detach mechanism 140 may comprise an electrolytic element 145 connecting the flexible joint 130 and the delivery element 150. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110. It is noted that in some embodiments, the flexible material 310 may be formed of the electrolytic material 145, the flexible joint 130 thus acting as both the flexible joint 130 and the detach mechanism 140.

The deployment method disclosed with respect to Figs. 2A to 2C can be used for the embolization system 100 shown in Fig. 6C. The flexible material 310 allows the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

Fig. 6D shows another embodiment of the embolization system described with respect to Fig. 1. In the embodiment of Fig. 6D, the flexible joint 130 is provided proximally to the detach mechanism 140. The flexible joint 130 may comprise a flexible material 310 (for example a wire, thread or ribbon). The flexible material 310 may be made of any suitable material such as a polymer or metal. The flexible material 310 is connected to the delivery element 150 at a proximal end and the detach mechanism 140 at a distal end. The flexible material 310 may be crimped to the detach mechanism 140 and the delivery element 150 by crimped hypotubes 312 or by adhesive or welding. Detach mechanism 140 may comprise an electrolytic element 145 connecting the flexible joint 130 and the embolization device 110. The flexible material 310 may be formed on the electrolytic element 145 or may itself be made of electrolytic material such that it acts as both the joint 130 and the electrolytic element 145. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The flexible material 310 may be electrically conductive itself or a flexible conductive wire may extend across the joint 130 to electrically connect the electrolytic element 145. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110.

The deployment method disclosed with respect to Figs. 2A to 2C can be used for the embolization system 100 shown in Fig. 6D. The flexible material 310 allows the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

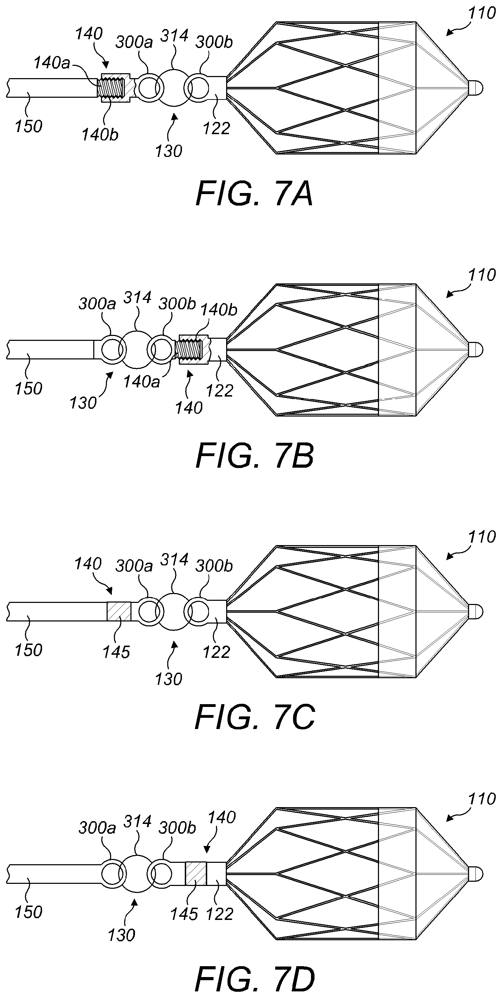

Fig. 7A shows an embodiment of the embolization system 100 described with respect to Fig. 1. In the embodiment of Fig. 7A, the flexible joint 130 is provided distally to the detach mechanism 140. The flexible joint 130 may comprise an articulating joint, which comprises an interlocking chain of loops 300a, 300b and 314. The interlocking chain of loops 300a, 300b and 314 may be made of any suitable material such as a polymer or metal. Loop 300b is connected to the embolization device 110 and loop 300a is connected to the detach mechanism 140. Loops 300a and 300b are connected to each other via intermediate loop 314. Detach mechanism 140 may comprise a female screw thread 140b which is configured to connect to a male screw thread 140a on a delivery element 150 (in other embodiments the detach mechanism 140 comprises a male screw thread connected to the flexible joint 130, configured to connect to a female screw thread on the delivery element 150). The loop 300b may be formed on the proximal fixing element 122 or may be formed directly on the skeleton 114 of embolization device 110.

The deployment method disclosed with respect to Figs. 2A to 2C can be used for the embolization system 100 shown in Fig. 7A. The interlocking loops 300a, 300b and 314 allow the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 7B shows another embodiment of the embolization system 100 described with respect to Fig. 1. In the embodiment of Fig. 7B, the flexible joint 130 is provided proximally to the detach mechanism 140. The flexible joint 130 may comprise an articulating joint, which comprises an interlocking chain of loops 300a, 300b and 314. The interlocking chain of loops 300a, 300b and 314 may be made of any suitable material such as a polymer or metal. Loop 300b is connected to the detach mechanism 140 and loop 300a is connected to the delivery element 150. Detach mechanism 140 may comprise a female screw thread 140b connected to embolization device 110 which is configured to connect to a male screw thread 140a connected to loop 300b (in other embodiments the detach mechanism 140 comprises a male screw thread connected to the embolization device 110, configured to connect to a female screw thread connected to the loop 300b). the female screw thread 140b may be formed on the proximal fixing element 122 or may be formed directly on the skeleton 114 of embolization device 110.

The deployment method disclosed with respect to Figs. 2A to 2C can be used for the embolization system 100 shown in Fig. 7B. The interlocking loops 300a, 300b and 314 allow the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 7C shows another embodiment of the embolization system described with respect to Fig. 1. In the embodiment of Fig. 7C, the flexible joint 130 is provided distally to the detach mechanism 140. The flexible joint 130 may comprise an articulating joint, which comprises interlocking loops 300a, 300b and 314. The interlocking chain of loops 300a, 300b and 314 may be made of any suitable material such as a polymer or metal. Loop 300b is connected to the embolization device 110 and loop 300a is connected to the detach mechanism 140. The loop 300b may be formed on the proximal fixing element 122 or may be formed directly on the skeleton 114 of embolization device 110. Detach mechanism 140 may comprise an electrolytic element 145 connecting the flexible joint 130 and the delivery element 150. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110. It is noted that in some embodiments, loops 300a, 300b and/or 314 may be formed of the electrolytic material 145, thus acting as both the flexible joint 130 and the detach mechanism 140.

The deployment method disclosed with respect to Figs. 2A to 2C can be used for the embolization system 100 shown in Fig. 7C. The interlocking loops 300a, 300b and 314 allow the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

Fig. 7D shows another embodiment of the embolization system described with respect to Fig. 1. In the embodiment of Fig. 7D, the flexible joint 130 is provided proximally to the detach mechanism 140. The flexible joint 130 may comprise an articulating joint, which comprises interlocking loops 300a, 300b and 314. The interlocking chain of loops 300a, 300b and 314 may be made of any suitable material such as a polymer or metal. Detach mechanism 140 may comprise an electrolytic element 145 connecting the flexible joint 130 and the embolization device 110. Loop 300b may be formed on the electrolytic element 145 or one or more of loops 300a, 300b, 314 may themselves be made of electrolytic material such that it acts as both the joint 130 and the electrolytic element 145. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The loops 300a, 300b and 314 may be electrically conductive themselves or a flexible conductive wire may extend across the joint 130 to electrically connect the electrolytic element 145. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110.

The deployment method disclosed with respect to Figs. 2A to 2C can be used for the embolization system 100 shown in Fig. 7D. The interlocking loops 300a, 300b and 314 allow the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

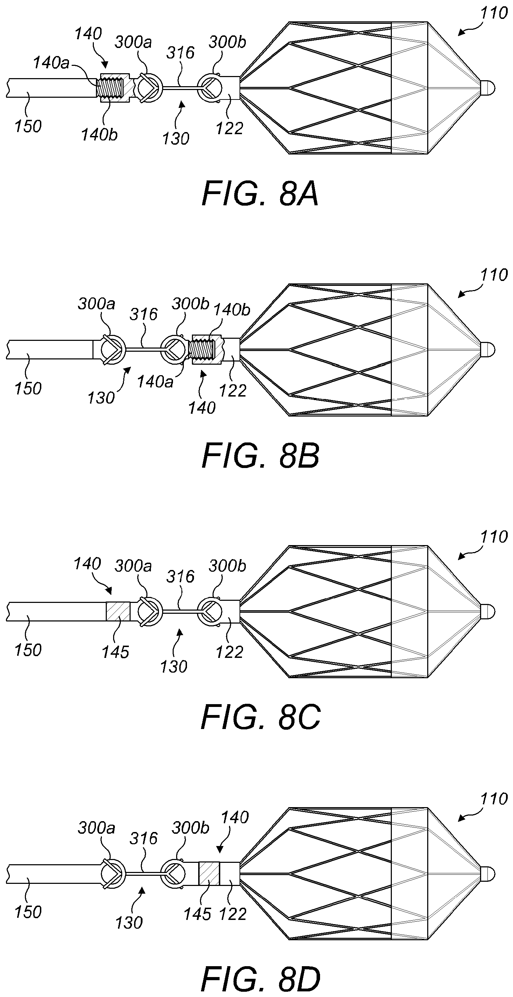

Fig. 8A shows an embodiment of the embolization system 100 described with respect to Fig. 1. In the embodiment of Fig. 8A, the flexible joint 130 is provided distally to the detach mechanism 140. The flexible joint 130 may comprise an articulating joint, which comprises loops 300a and 300b connected via a connecting piece 316 comprising connecting arms which inhibit the loops 300a and 300b from being separated. The loops 300a, 300b and connecting piece 316 may be made of any suitable material such as a polymer or metal. Loop 300b is connected to the embolization device 110 and loop 300a is connected to the detach mechanism 140. Detach mechanism 140 may comprise a female screw thread 140b which is configured to connect to a male screw thread 140a on a delivery element 150 (in other embodiments the detach mechanism 140 comprises a male screw thread connected to the flexible joint 130, configured to connect to a female screw thread on the delivery element 150). The loop 300b may be formed on the proximal fixing element 122 or may be formed directly on the skeleton 114 of embolization device 110.

The deployment method disclosed with respect to Figs. 2A to 2C can be used for the embolization system 100 shown in Fig. 8A. The interlocking loops 300a, 300b and connecting piece 316 allow the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 8B shows another embodiment of the embolization system 100 described with respect to Fig. 1. In the embodiment of Fig. 8B, the flexible joint 130 is provided proximally to the detach mechanism 140. The flexible joint 130 may comprise an articulating joint, which comprises loops 300a and 300b connected via a connecting piece 316 comprising connecting arms which inhibit the loops 300a and 300b from being separated. The loops 300a, 300b and connecting piece 316 may be made of any suitable material such as a polymer or metal. Loop 300b is connected to the detach mechanism 140 and loop 300a is connected to the delivery element 150. Detach mechanism 140 may comprise a female screw thread 140b connected to embolization device 110 which is configured to connect to a male screw thread 140a connected to loop 300b (in other embodiments the detach mechanism 140 comprises a male screw thread connected to the embolization device 110, configured to connect to a female screw thread connected to the loop 300b). the female screw thread 140b may be formed on the proximal fixing element 122 or may be formed directly on the skeleton 114 of embolization device 110.

The deployment method disclosed with respect to Figs. 2A to 2C can be used for the embolization system 100 shown in Fig. 8B. The interlocking loops 300a, 300b and connecting piece 316 allow the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 8C shows another embodiment of the embolization system described with respect to Fig. 1. In the embodiment of Fig. 8C, the flexible joint 130 is provided distally to the detach mechanism 140. The flexible joint 130 may comprise an articulating joint, which comprises loops 300a and 300b connected via a connecting piece 316 comprising connecting arms which inhibit the loops 300a and 300b from being separated. The loops 300a, 300b and connecting piece 316 may be made of any suitable material such as a polymer or metal. Loop 300b is connected to the embolization device 110 and loop 300a is connected to the detach mechanism 140. The loop 300b may be formed on the proximal fixing element 122 or may be formed directly on the skeleton 114 of embolization device 110. Detach mechanism 140 may comprise an electrolytic element 145 connecting the flexible joint 130 and the delivery element 150. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110. It is noted that in some embodiments, loops 300a, 300b and/or connecting piece 316 may be formed of the electrolytic material 145, thus acting as both the flexible joint 130 and the detach mechanism 140.

The deployment method disclosed with respect to Figs. 2A to 2C can be used for the embolization system 100 shown in Fig. 8C. The interlocking loops 300a, 300b and connecting piece 316 allow the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

Fig. 8D shows another embodiment of the embolization system described with respect to Fig. 1. In the embodiment of Fig. 8D, the flexible joint 130 is provided proximally to the detach mechanism 140. The flexible joint 130 may comprise an articulating joint, which comprises loops 300a and 300b connected via a connecting piece 316 comprising connecting arms which inhibit the loops 300a and 300b from being separated. The loops 300a, 300b and connecting piece 316 may be made of any suitable material such as a polymer or metal. Detach mechanism 140 may comprise an electrolytic element 145 connecting the flexible joint 130 and the embolization device 110. Loop 300b may be formed on the electrolytic element 145 or one or more of loops 300a, 300b, and connecting piece 316 may themselves be made of electrolytic material such that it acts as both the joint 130 and the electrolytic element 145. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The loops 300a, 300b and connecting piece 316 may be electrically conductive themselves or a flexible conductive wire may extend across the joint 130 to electrically connect the electrolytic element 145. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110.

The deployment method disclosed with respect to Figs. 2A to 2C can be used for the embolization system 100 shown in Fig. 8D. The interlocking loops 300a, 300b and connecting piece 316 allow the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

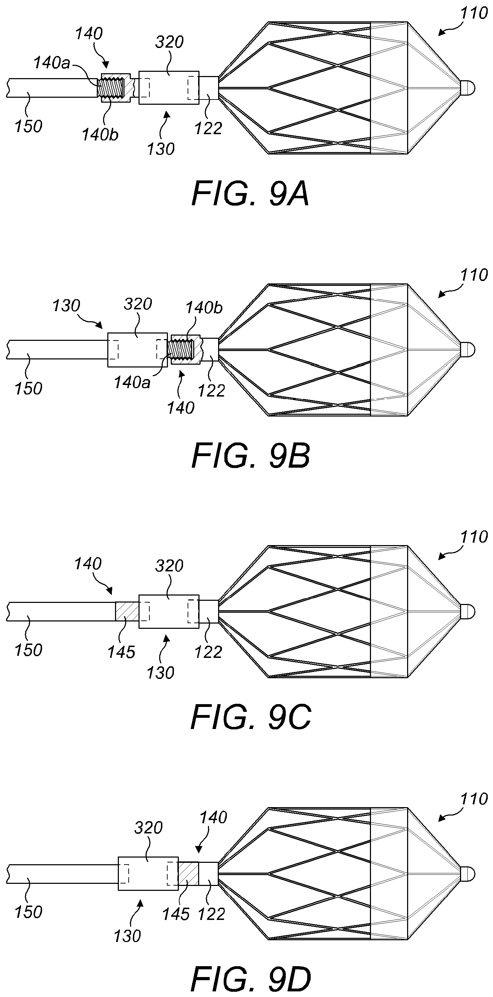

Fig. 9A shows an embodiment of the embolization system 100 described with respect to Fig. 1. In the embodiment of Fig. 9A, the flexible joint 130 is provided distally to the detach mechanism 140. The flexible joint 130 may comprise a flexible tube 320. The flexible tube 320 may be made of any suitable material such as a polymer or metal. The flexible tube 320 is connected to the detach mechanism 140 at a proximal end and the embolization device 110 (proximal fixing element 122 or skeleton 112) at a distal end. The flexible tube 320 may be made of, for example, a heat-shrinkable material and heat-shrunk a distal end of the detach mechanism 140 and a proximal end of the embolization device 110. Alternatively, the flexible tube 320 may be attached by adhesive or welding. Detach mechanism 140 may comprise a female screw thread 140b which is configured to connect to a male screw thread 140a on a delivery element 150 (in other embodiments the detach mechanism 140 comprises a male screw thread connected to the flexible joint 130, configured to connect to a female screw thread on the delivery element 150).

The deployment method disclosed with respect to Figs. 2A to 2C can be used for the embolization system 100 shown in Fig. 9A. Flexible tube 320 allows the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 9B shows an embodiment of the embolization system 100 described with respect to Fig. 1. In the embodiment of Fig. 9B, the flexible joint 130 is provided proximally to the detach mechanism 140. The flexible joint may comprise a flexible tube 320. The flexible tube 320 may be made of any suitable material such as a polymer or metal. The flexible tube 320 is connected to the delivery element 150 at a proximal end and the detach mechanism 140 at a distal end. The flexible tube 320 may be made of, for example, a heat-shrinkable material and heat-shrunk a distal end of the delivery element 150 and a proximal end of the detach mechanism 140. Alternatively, the flexible tube 320 may be attached by adhesive or welding. Detach mechanism 140 may comprise a female screw thread 140b formed on the proximal fixing element 122 or directly on the skeleton 114, which is configured to connect to a male screw thread 140a connected to flexible joint 130 (in other embodiments the detach mechanism 140 comprises a male screw thread connected to the embolization device 110, configured to connect to a female screw thread connected to the delivery element 150 via flexible joint 130).

The deployment method disclosed with respect to Figs. 2A to 2C can be used for the embolization system 100 shown in Fig. 9B. The flexible tube 320 allows the embolization device 110 to tilt with respect to the delivery element 150 in configurations such as that shown in Fig. 2B. When the embolization device 110 is correctly positioned in the bodily lumen, the delivery element 150 may be rotated to release the embolization device 110 from the delivery element 150 and the delivery element 150 and delivery catheter 200 can be removed from the bodily lumen as illustrated in Fig. 2C.

Fig. 9C shows an embodiment of the embolization system 100 described with respect to Fig. 1. In the embodiment of Fig. 9C, the flexible joint 130 is provided distally to the detach mechanism 140. The flexible joint 130 may comprise a flexible tube 320. The flexible tube 320 may be made of any suitable material such as a polymer or metal. The flexible tube 320 is connected to the detach mechanism 140 at a proximal end and the embolization device 110 (proximal fixing element 122 or skeleton 112) at a distal end. The flexible tube 320 may be made of, for example, a heat-shrinkable material and heat-shrunk a distal end of the detach mechanism 140 and a proximal end of the embolization device 110. Alternatively, the flexible tube 320 may be attached by adhesive or welding. Detach mechanism 140 may comprise an electrolytic element 145 connecting the flexible joint 130 and the delivery element 150. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110. It is noted that in some embodiments, the flexible tube 320 may be formed at least partially of the electrolytic material 145 (for example the flexible tube 320 may be made of the electrolytic material 145 and comprise a plurality of slots in the tube 320 to facilitate bending of the tube), the flexible joint 130 thus acting as both the flexible joint 130 and the detach mechanism 140.

The deployment method disclosed with respect to Figs. 2A to 2C can be used for the embolization system 100 shown in Fig. 9C. The flexible tube 320 allows the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

Fig. 9D shows another embodiment of the embolization system described with respect to Fig. 1. In the embodiment of Fig. 9D, the flexible joint 130 is provided proximally to the detach mechanism 140. The flexible joint 130 may comprise a flexible tube 320. The flexible tube 320 may be made of any suitable material such as a polymer or metal. The flexible tube 320 is connected to the delivery element 150 at a proximal end and the detach mechanism 140 at a distal end. The flexible tube 320 may be made of, for example, a heat-shrinkable material and heat-shrunk a distal end of the delivery element 150 and a proximal end of the detach mechanism 140. Alternatively, the flexible tube 320 may be attached by adhesive or welding. Detach mechanism 140 may comprise an electrolytic element 145 connecting the flexible joint 130 and the embolization device 110. The flexible tube 320 may be formed on the electrolytic element 145 or may itself be made of electrolytic material as in the case of the embodiment described with respect to Fig. 9C, such that it acts as both the joint 130 and the electrolytic element 145. The electrolytic element 145 is electrically connected to a proximal end of the embolization system 100, and is operable to disintegrate by electrolysis in the body lumen to detach the embolization device 110 from the delivery element 150. By applying an electric current to the electrolytic element, at a current amplitude, a voltage and for a duration of time, such that the electrical energy supplied to the electrolytic element is above a disintegration energy of the electrolytic element, the delivery element 150 is detached from the embolization device 110. The delivery element 150 may be electrically conductive itself or a conductive wire (not shown) may be provided that runs along the length of the delivery element 150. The flexible tube 320 may be electrically conductive itself or a flexible conductive wire may extend across the joint 130 to electrically connect the electrolytic element 145. The electrolytic element 145 may be formed of any material configured to disintegrate by electrolysis in the body lumen upon application of an electric current. Suitable materials include platinum, stainless steel, nitinol and cobalt chromium. The electric current applied may be a positive direct current. A corresponding electrode may be provided proximal to the location of the embolization device 110 to complete the circuit, for example inside the bodily lumen adjacent the electrolytic element or on the surface of the patient proximal to the location of the embolization device 110.

The deployment method disclosed with respect to Figs. 2A to 2C can be used for the embolization system 100 shown in Fig. 9D. The flexible tube 320 allows the embolization device 110 to tilt with respect to the delivery element 150 in configurations such as that shown in Fig. 2B. When the embolization device 110 is correctly positioned in the bodily lumen, electric current is applied to the electrolytic element 145 to release the embolization device 110 from the delivery element 150 and the delivery element 150 and delivery catheter 200 can be removed from the bodily lumen as illustrated in Fig. 2C.

In embodiments where the detach mechanism is provided distally to the flexible joint, advantageously the flexible joint is removed from the bodily lumen upon deployment, which may be beneficial if the flexible joint has a risk of detaching from the embolization device when deployed in the lumen.

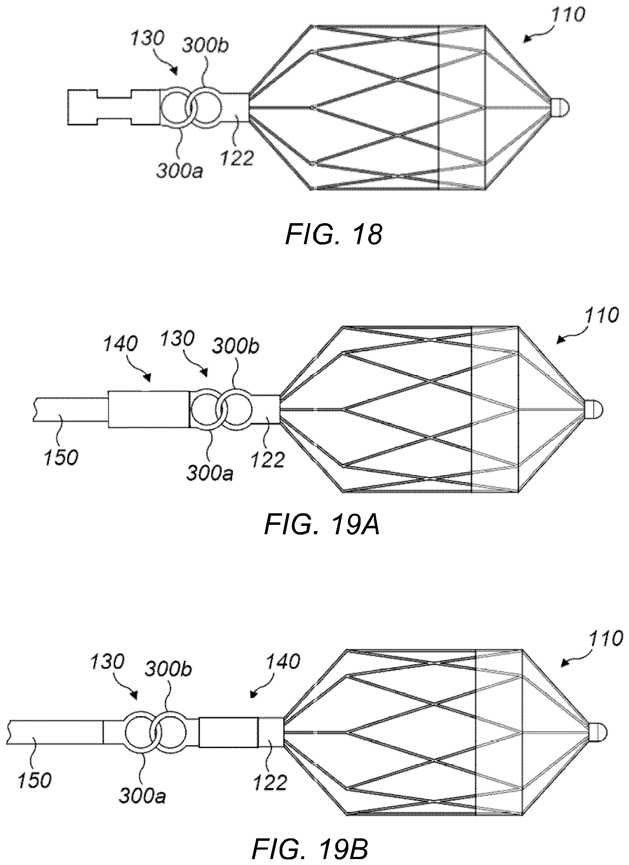

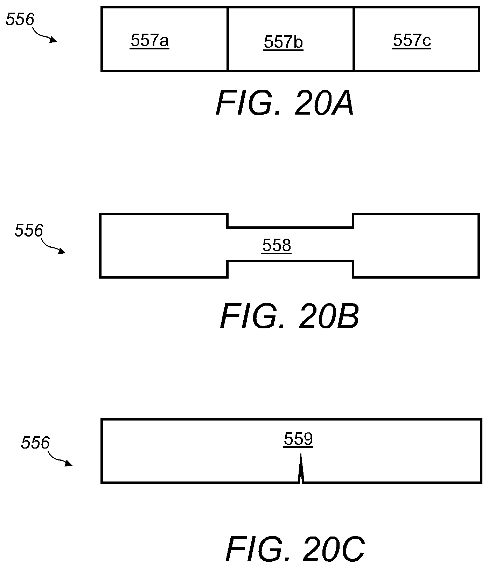

Fig. 19A shows an embodiment of the embolization system 100 described with respect to Fig. 1. In the embodiment of Fig. 19A, the flexible joint 130 is provided distally to the detach mechanism 140. In the illustrated embodiment, the flexible joint 130 may comprise an articulating joint, which comprises interlocking loops 300a and 300b. In other embodiments, the flexible joint 130 may comprise any of the flexible joints 130 described with reference to Figs. 4A to 9D. Loop 300b is connected to the embolization device 110 and loop 300a is connected to the detach mechanism 140. The interlocking loops 300a, 300b may be made of any suitable material such as a polymer or metal. The loop 300b may be formed on the proximal fixing element 122 or may be formed directly on the skeleton 112 of embolization device 110. Detach mechanism 140 may include a breakable detachment element 556 connecting a proximal portion of flexible joint 130 (in the illustrated embodiment loop 300a) and delivery element 150. The breakable detachment element 556 may be fixed to the proximal portion of flexible joint 130 and distal portion of delivery element 150 by any suitable attachment means, such as by welding, adhesive or crimping. The breakable detachment element 556 may be any suitable breakable detachment element 556, such as any of those described with reference to Figs. 20A to 20C.

The deployment method disclosed with respect to Figs. 2A to 2C can be used for the embolization system 100 shown in Fig. 19A. The interlocking loops 300a and 300b allow the embolization device 110 to tilt with respect to the delivery element 150 in configurations such as that shown in Fig. 2B. When the embolization device 110 is correctly positioned in the bodily lumen, the delivery element 150 may be rotated by a predetermined amount, in either direction, about the longitudinal axis of the delivery element 150. The rotation can be effected by a user at the proximal end of the delivery wire, manually or by using any suitable rotation mechanism. As the embolization device 110 is anchored to the bodily lumen, relative rotation between the delivery element 150 and the embolization device 110 occurs. This rotation results in an increased amount of torque being applied at the detachment mechanism 140. The breakable detachment element 556 is configured to break preferentially before the delivery element 150 nor the flexible joint 130 breaks. Once sufficient torque is applied to the detachment mechanism 140, the breakable detachment element 556 breaks. In any of the embodiments disclosed herein, the force required to break the breakable detachment element 556 can be selected to be low enough such that the embolization device 110 is not dislodged or moved from the anchored position in the bodily lumen when applying the force to break the breakable detachment element 556. This breaking force can be selected based on the anchoring properties of the particular embolization device 110 being used.

Fig. 19B shows an embodiment of the embolization system 100 described with respect to Fig. 1. In the embodiment of Fig. 19B, the flexible joint 130 is provided distally to the detach mechanism 140. In the illustrated embodiment, the flexible joint 130 may comprise an articulating joint, which comprises interlocking loops 300a and 300b. In other embodiments, the flexible joint 130 may comprise any of the flexible joints 130 described with reference to Figs. 4A to 9D. Loop 300b is connected to the embolization device 110 and loop 300a is connected to the detach mechanism 140. The interlocking loops 300a, 300b may be made of any suitable material such as a polymer or metal. The loop 300b may be formed on the proximal fixing element 122 or may be formed directly on the skeleton 112 of embolization device 110. Detach mechanism 140 may include a breakable detachment element 556 connecting a proximal portion of embolization device 110 and a distal portion of the flexible joint 130 (in the illustrated embodiment loop 300b). The breakable detachment element 556 may be fixed to the proximal portion of embolization device 110 and distal portion of the flexible joint 130 by any suitable attachment means, such as by welding, adhesive or crimping. The breakable detachment element 556 may be any suitable breakable detachment element 556, such as any of those described with reference to Figs. 20A to 20C.

The deployment method disclosed with respect to Figs. 2A to 2C can be used for the embolization system 100 shown in Fig. 19A. The interlocking loops 300a and 300b allow the embolization device 110 to tilt with respect to the delivery element 150 in configurations such as that shown in Fig. 2B. When the embolization device 110 is correctly positioned in the bodily lumen, the delivery element 150 may be rotated by a predetermined amount, in either direction, about the longitudinal axis of the delivery element 150. The rotation can be effected by a user at the proximal end of the delivery wire, manually or by using any suitable rotation mechanism. As the embolization device 110 is anchored to the bodily lumen, relative rotation between the delivery element 150 and the embolization device 110 occurs. This rotation results in an increased amount of torque being applied at the detachment mechanism 140 (the rotation of the delivery element 150 is transmitted to the breakable detachment element by the flexible joint 130). The breakable detachment element 556 is configured to break preferentially before the delivery element 150 nor the flexible joint 130 breaks. Once sufficient torque is applied to the detachment mechanism 140, the breakable detachment element 556 breaks. In any of the embodiments disclosed herein, the force required to break the breakable detachment element 556 can be selected to be low enough such that the embolization device 110 is not dislodged or moved from the anchored position in the bodily lumen when applying the force to break the breakable detachment element 556. This breaking force can be selected based on the anchoring properties of the particular embolization device 110 being used.

It is noted that whilst in the embodiments described with reference to Figs. 19A and 19B (as well as Figs. 3A to 9D) the flexible joint 130 and detach mechanism 140 are provided as separate elements, it may be that the delivery element 150 and the proximal end of the embolization device 110 are connected by a singular breakable detachment element 556 which is also flexible such that it additionally acts flexible joint 130, i.e. having a higher flexibility than the embolization device for allowing the embolization device to tilt with respect to the delivery element when the delivery element is connected to the embolization device. In such embodiments, the breakable detachment element 556 directly connects the delivery element 150 and the proximal end of the embolization device 110. It may be preferred to provide the flexible joint 130 and detach mechanism 140 as separate elements, wherein the flexible joint 130 is configured to bend more than detach mechanism 140 when a bending force is applied to the embolization system, so that the detach mechanism 140 does not break prematurely.

Fig. 20A shows a breakable detachment element 556 for an embolization system according to one or more embodiments. The breakable detachment element 556 of FIG. 20A may comprise a portion 557b that is a different material from the material of both proximal element 557a and distal element 557c which the breakable detachment element 556 connects (for example, in the illustrated embodiment of Fig. 19A, the proximal element 557a is the delivery element 150 and the distal element 557c is proximal portion of flexible joint 130; in the illustrated embodiment of Fig. 19B, the proximal element 557a is the distal portion of flexible joint 130 and the distal element 557c is a proximal portion of the embolization device 110). In particular, the material of portion 557b may be selected to be a material which is less stiff than the material of the proximal element 557a and the distal element 557c. As such, any torque applied to the system (for example at delivery element 150) results in a larger amount of twist at the portion 557b. Further, when provided as separate elements, the material of portion 557a may be selected so that the torque required to break the portion 557a is lower than the torque required to break the flexible joint 130. After a sufficient amount of torque is applied to the delivery wire 150, the portion 557b breaks and the stem 110 is separated from the delivery wire 150. The amount of torque required to break the portion 557b (i.e. the amount of rotation of the delivery wire 150) may be determined by the material properties of the portion 557b, the proximal element 557a and the distal element 557c. The materials can be selected such that the portion 557b is configured to shear upon an amount of rotation of the delivery element 150 that is above the expected amount of relative rotation of the device during the delivery process (due to the tortuous path taken by the device through the delivery catheter). For example, the material of the portion 557b may be selected from Nylon, PTFE, or Cobalt-chrome, and the materials of the proximal element 557a and the distal element 557c may be selected from nitinol or Cobalt-chrome (such that the material of the portion 557b differs from both).

Fig. 20B shows a breakable detachment element 556 according to one or more embodiments. The detachment element 556 of FIG. 20B may comprise a necked portion 558. The necked portion 558 may have a radial extent selected such that the necked portion preferentially breaks when a torque is applied to the embolization system via delivery element 150. The necked portion may have a radial extent that is less than the radial extent of the delivery element 150. The necked portion is prone to a high amount of twist when torque is applied to the delivery element 150. As a result, after a sufficient amount of torque is applied to the delivery element 150, the necked portion 558 breaks and the embolization device 110 is separated from the delivery element 150. The amount of torque required to break the necked portion 558 (i.e. the amount of rotation of the delivery element 150) may be determined by the dimensions of the necked portion 558 and the material properties of the necked portion 558. The dimensions and material properties can be selected such that the necked portion 558 is configured to shear upon an amount of rotation of the delivery element 150 that is above the expected amount of relative rotation of the device during the delivery process (due to the tortuous path taken by the device through the delivery catheter). In embodiments where the flexible joint 130 is provided as a separate element, the shear force required to break the necked portion 558 is also higher than the shear force required to break the flexible joint 130. For example, the detachment element may be made of Nylon, PTFE, or Cobalt-chrome and a cross-sectional area of the necked portion may be selected to be 50% or less of the cross-sectional area of the delivery element 150. The necked portion 558 may be provided in an element which is then connected to the relevant proximal and distal elements (for example, in the case of embodiment such as Fig. 19A, the delivery element 150 and proximal portion of flexible joint 130; in the case of embodiments such as Fig. 19B, the distal portion of flexible joint 130 and proximal portion of embolization device 110). In other embodiments where the detach mechanism 140 is provided proximal to the flexible joint 130 (e.g. Fig. 19A) or where the breakable detach element 556 is also the flexible joint 130, the necked portion 558 may be provided directly on the delivery element 150 (for example machined or otherwise formed on delivery element 150) and the delivery element 150 may be connected directly to the flexible joint 130 (or embolization device 110 where the breakable detach mechanism is also the flexible joint 130).

Fig. 20C shows a breakable detachment element 556 according to one or more embodiments. The breakable detachment element 556 of FIG. 20C may comprise a weakening structure 559. The weakening structure 559 may comprise an irregularity such that the shearing force required to break it is lower than the shearing force required to break the other elements of the embolization system. As illustrated in FIG. 20C, the weakening structure 559 may be a fracture. The fracture may have a radial extent that is less than the radial extent of the delivery element 150. The weakening structure 559 is prone to a high amount of twist when torque is applied to the delivery element 150. As a result, after a sufficient amount of torque is applied to the delivery element 150, the weakening structure 559 breaks and the embolization device 110 is separated from the delivery element 150. The amount of torque required to break the weakening structure 559 (i.e. the amount of rotation of the delivery element 150) may be determined by the dimensions of the fracture. The relative dimensions can be selected such that the weakening structure 559 is configured to shear upon an amount of rotation of the delivery wire 150 that is above the expected amount of relative rotation of the device during the delivery process (due to the tortuous path taken by the device through the delivery catheter). The weakening structure may be configured to preferential break before the other elements of the embolization system (i.e. at a shear force which is lower than the shear force required to break the other elements of the embolization system). In embodiments where the flexible joint 130 is provided as a separate element, the shear force required to break the breakable detachment element 556 is lower than that of the flexible joint 130. The detachment element may be made of Nylon, PTFE, or Cobalt-chrome and may be the same or different material to the delivery element 150 and/or flexible joint 130 and/or embolization device 110. A cross-sectional area of the breakable detachment element 559 may be selected to be 50% or less of the cross-sectional area of the delivery element 150. The weakening structure 559 may be provided on an element which is then connected to the relevant proximal and distal elements (for example, in the case of embodiment such as Fig. 19A, the delivery element 150 and proximal portion of flexible joint 130; in the case of embodiments such as Fig. 19B, the distal portion of flexible joint 130 and proximal portion of embolization device 110). In other embodiments where the detach mechanism 140 is provided proximal to the flexible joint 130 (e.g. Fig. 19A) or where the breakable detach element 556 is also the flexible joint 130, the weakening structure 559 may be provided directly on the delivery element 150 (for example machined or otherwise formed on delivery element 150) and the delivery element 150 may be connected directly to the flexible joint 130 or embolization device 110 (or embolization device 110 where the breakable detach mechanism is also the flexible joint 130).

In some embodiments, the breakable detachment element 556 may comprise any two or all three of the portion 557b, necked portion 558 and weakening structure 559.

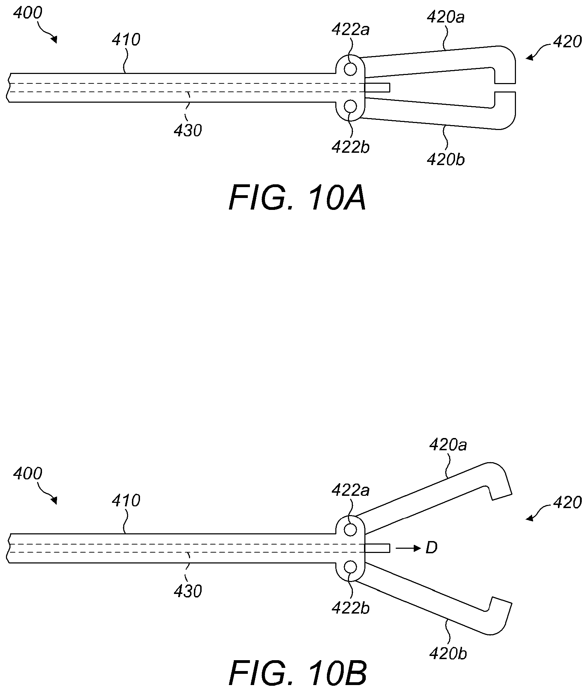

In any of the embodiments of the embolization system, the detach mechanism may instead be comprised in any of the delivery systems disclosed herein with reference to Figs. 10A to 17B.

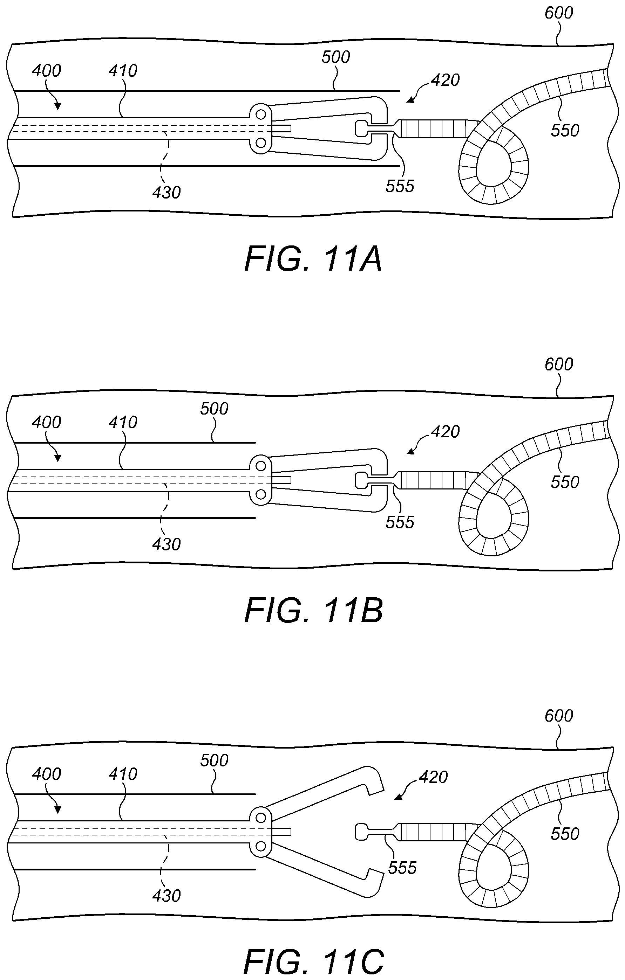

Fig. 10A shows a delivery system 400 for delivering and deploying an implant, such as the embolization device 110 described with reference to Fig. 1, an embolization device such as that described in European patents EP 2 967 569 B1 or EP 3 193 743 B1, or am embolization coil, to a bodily lumen according to one or more embodiments. The delivery system may generally include a delivery element 410 configured to extend through a lumen of a delivery catheter, a detach mechanism 420 mounted to a distal portion of the delivery element 410, and an actuating mechanism 430. The delivery element 410, detach mechanism 420 and actuating mechanism 430 may be made of any suitable material such as a polymer or metal. The detach mechanism 420 has a first configuration in which the detach mechanism 420 is configured to grip the implant, as shown in Fig. 10A and Figs. 11A and 11B, and a second configuration in which the detach mechanism 420 is configured to release the implant, as shown in Fig. 10B and Fig. 11C. The actuating mechanism 430 is configured to extend through the lumen of the delivery catheter to the detach mechanism 420. In Fig. 10A the actuating mechanism 430 extends through a lumen of the delivery element 410. The delivery element 410 prevents the actuating mechanism 420 from buckling. In other embodiments, the actuating mechanism 420 may be external to the delivery element 410. The actuating mechanism 420 is movable between a first position and a second position. The first position is shown in Fig. 10A.

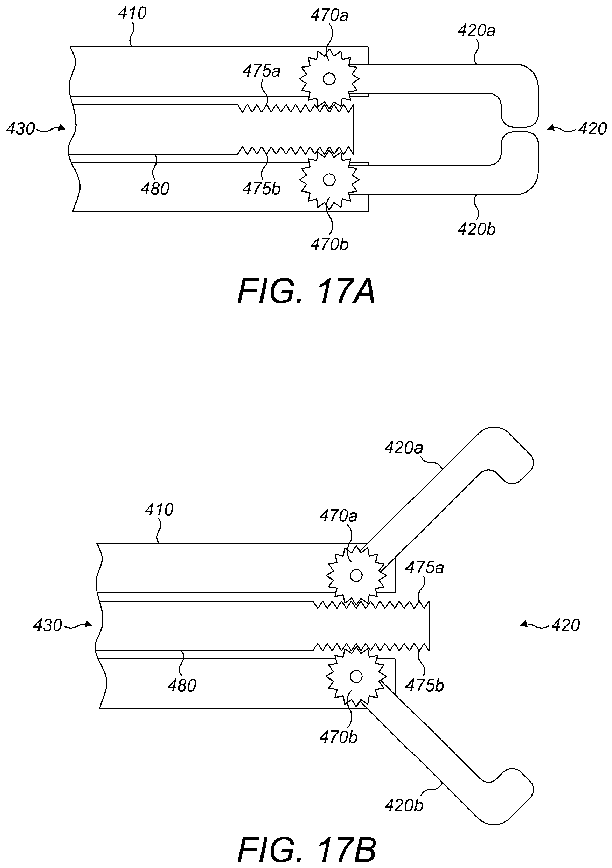

Fig. 10B shows the delivery system 400 of Fig. 10A when the actuating mechanism 420 is in the second position. Moving the actuating mechanism 420 from the first position to the second position (i.e. in direction D) changes the detach mechanism 420 from the first configuration to the second configuration. In the particular embodiment shown in Figs. 10A and 10B, the detach mechanism 420 comprises a pair of gripping elements 420a, 420b hingedly mounted to delivery element 410 via hinges 422a, 422b. The actuating mechanism 420 is coupled to the gripping elements 420a, 420b such that movement of the actuating mechanism 420 distally to the second position causes the gripping elements 420a, 420b to pivot about hinges 422a, 422b so that they open. The actuating mechanism 420 may be coupled to the gripping elements 420a, 420b by friction or the actuating mechanism 420 and gripping elements 420a, 420b may comprises interlocking teeth 470a, 470b, 475a, 475b (as illustrated in Figs. 17A and 17B) which engage so that movement of the actuating mechanism 420 results in the pivoting motion of the gripping elements 420a, 420b. The gripping elements 420a, 420b may be claws comprising inwardly facing teeth in order to assist in gripping the medical implant.

Fig. 11A shows the delivery system 400 in a delivery catheter 500 in a bodily lumen 600. The delivery system is in the first position and grips a proximal end of a medical implant 550, which in the illustrated embodiment is an embolization coil. The medical implant 550 may comprise a gripping feature 555 having a recess which is configured to receive the inwardly facing teeth of the gripping elements 420a, 420b inhibit premature release of the medical implant 550 from the delivery system 400. In the illustrated embodiment of Fig. 11A, the delivery system 400 has been pushed through the delivery catheter to a distal tip of the delivery catheter 500 so that the medical implant 550 has been delivered into the bodily lumen 600 from the distal tip of the delivery catheter. The medical implant 550 remains gripped by the delivery system 400 so that it can still be retrieved back into the delivery catheter 500 by moving the delivery system 400 proximally relative to the delivery catheter 500. The delivery catheter 500 may be sized so that the delivery system 400 is unable to move to the second position when the detach mechanism 420 is inside the delivery catheter 500 (i.e. the detach mechanism 420 abuts the inner walls of the delivery catheter 500 so that it is unable to move to the second position).

Fig. 11B shows the delivery system of Fig. 11A in a second configuration wherein the delivery catheter 500 and delivery system 400 have been moved with respect to each other (by moving the delivery system 400 distally relative to the delivery catheter 500 and/or moving the delivery catheter 500 proximally relative to the delivery system 400), so that the detach mechanism 420 is exterior to the distal tip of the delivery catheter 500. As such, the detach mechanism 420 is free to move from the first position to the second position.

Fig. 11C shows the delivery system 400 of Fig. 11B after the detach mechanism 420 is moved from the first position to the second position. A user of the delivery system 400 actuates a proximal end of the delivery catheter and/or actuating mechanism 420 which is outside of the body to provide the required movement from the first position to the second position. The medical implant 550 is no longer gripped by the delivery system 400. The delivery system 400 and delivery catheter 500 can be removed from the bodily lumen 600 and the medical implant 550 is deployed.

It is noted that the detach mechanism 420 could also be moved from the second position to the first position to re-capture medical implant 550 after deployment.

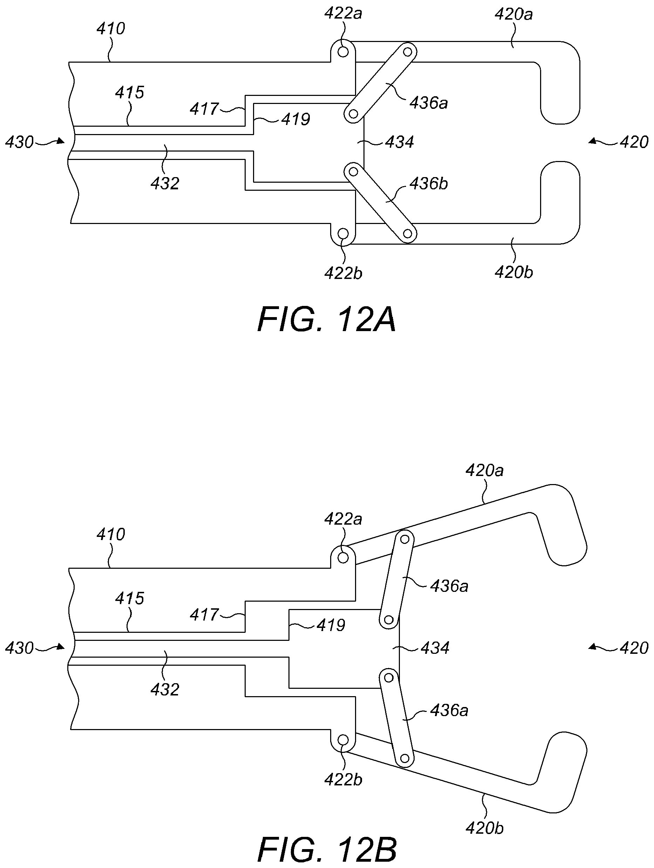

Fig. 12A shows a delivery system for delivering and deploying an implant to a bodily lumen according to one or more embodiments. The delivery system comprises a delivery element 410 configured to extend through a lumen of a delivery catheter, a detach mechanism 420 mounted to a distal portion of the delivery element 410, and an actuating mechanism 430. The delivery element 410, detach mechanism 420 and actuating mechanism 430 may be made of any suitable material such as a polymer or metal. The detach mechanism 420 has a first configuration in which the detach mechanism 420 is configured to grip the implant, as shown in Fig. 12A, and a second configuration in which the detach mechanism 420 is configured to release the implant, as shown in Fig. 12B. The actuating mechanism 430 extends through a lumen of the delivery element 410. The delivery element 410 prevents the actuating mechanism 420 from buckling. The actuating mechanism 420 is movable between a first position and a second position. The first position is shown in Fig. 12A.

The actuating mechanism 430 of Fig. 12A comprises an elongate element 432 (e.g. wire or ribbon) which extends through inner lumen 415 of the delivery element 410. The detach mechanism 420 comprises a pair of gripping elements 420a, 420b hingedly mounted to delivery element 410 via hinges 422a, 422b. The pair of gripping elements 420a, 420b are additionally hingedly connected to a first ends of links 436a, 436b respectively. The actuating mechanism 420 is hingedly mounted to second ends opposite the first ends of links 436a, 436b at distal part 434 such that movement of the actuating mechanism 420 distally to the second position causes the links 436a, 436b to transmit an opening force to the gripping elements 420a, 420b, moving the detach mechanism to the second position. Fig. 12B shows the delivery system in the second position. It is noted that when the delivery system is in the second position, movement of the actuating mechanism proximally or even further distally may move the gripping elements 420a, 420b back to the first position. The gripping elements 420a, 420b may be claws comprising inwardly facing teeth in order to assist in gripping the embolization device.

The distal part 434 may be shaped so that it does not fit inside inner lumen 415. The distal part 434 may comprise a first stopping element, namely a proximal shoulder 417 configured to abut with a corresponding stopping element, namely a distal shoulder 417 of delivery element 410 to form a stopper mechanism so that proximal translation of the actuating mechanism 430 relative to the delivery element 410 past a most proximal position is prevented. This may prevent damage to the gripped medical implant or the detach mechanism 420.

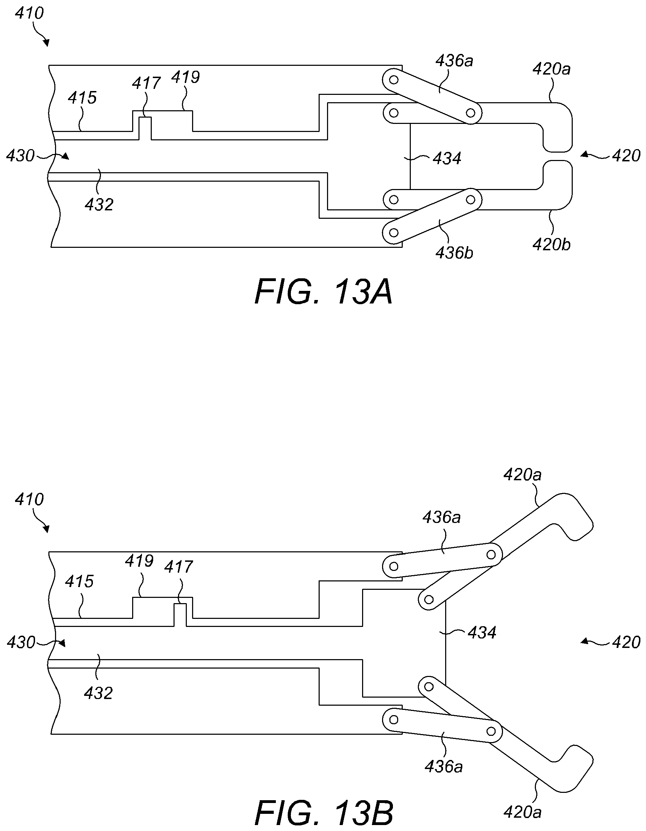

Fig. 13A shows a delivery system for delivering and deploying an implant to a bodily lumen according to one or more embodiments. The delivery system comprises a delivery element 410 configured to extend through a lumen of a delivery catheter, a detach mechanism 420 mounted to a distal portion of the delivery element 410, and an actuating mechanism 430. The delivery element 410, detach mechanism 420 and actuating mechanism 430 may be made of any suitable material such as a polymer or metal. The detach mechanism 420 has a first configuration in which the detach mechanism 420 is configured to grip the implant, as shown in Fig. 13A, and a second configuration in which the detach mechanism 420 is configured to release the implant, as shown in Fig. 13B. The actuating mechanism 430 extends through a lumen of the delivery element 410. The delivery element 410 prevents the actuating mechanism 420 from buckling. The actuating mechanism 420 is movable between a first position and a second position. The first position is shown in Fig. 13A.

The actuating mechanism 430 of Fig. 13A comprises an elongate element 432 (e.g. wire or ribbon) which extends through inner lumen 415 of the delivery element 410. The detach mechanism 420 comprises a pair of gripping elements 420a, 420b hingedly mounted to a distal part 434 of the actuating mechanism 430 and connected indirectly to delivery element 410. Delivery element 410 is hingedly connected to first ends of links 436a, 436b. The gripping elements 420a, 420b are hingedly connected to second ends of links 436a, 436b opposite the first ends. Movement of the actuating mechanism 430 distally relative to the delivery element 410 causes the gripping elements 420a, 420b to be pushed in a distal direction. Further, relative movement of the delivery element in a proximal direction (relative to the actuating mechanism 430) causes a pulling force to be exerted on the gripping elements 420a, 420b via links 436a, 436b. Accordingly, a pivoting force is exerted on the gripping elements 420a, 420b and the detach mechanism 420 moves to a second position as shown in Fig. 13B, for releasing a medical implant from the detach mechanism. The delivery element 410 and actuating mechanism 430 may together comprise a stopper mechanism formed by a first stopping element, namely recess 419 on one of the delivery element 419 and actuating mechanism 430 and a second stopping element, namely protrusion 417 on the other. The recess 419 receives the protrusion 417 so that relative displacement of the actuating mechanism beyond a most proximal and a most distal point is prevented. This may prevent damage to the detach mechanism 420 or medical implant when gripped.

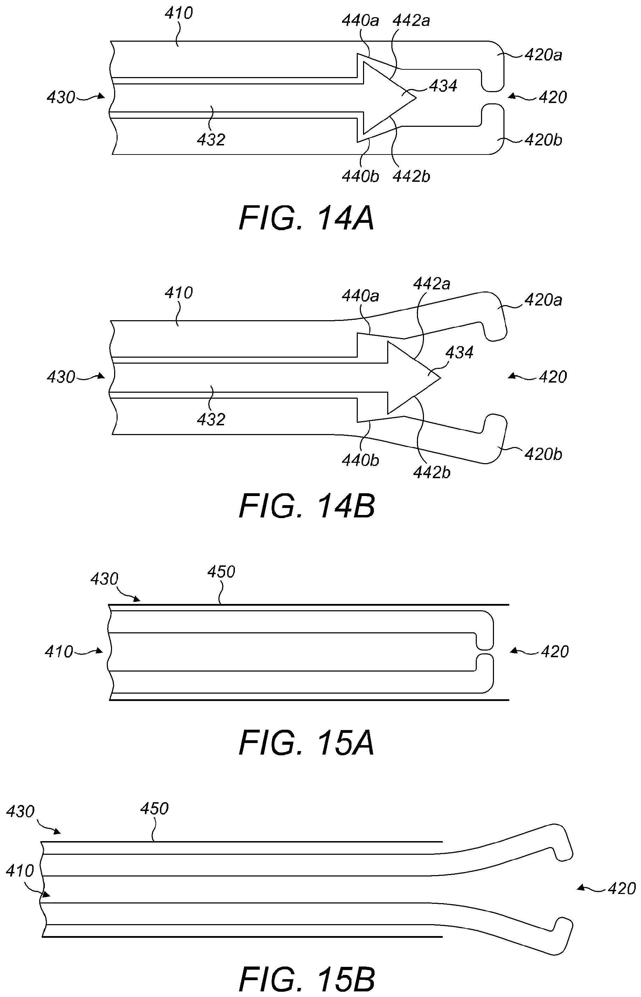

Fig. 14A shows a delivery system for delivering and deploying an implant to a bodily lumen according to one or more embodiments. The delivery system comprises a delivery element 410 configured to extend through a lumen of a delivery catheter, a detach mechanism 420 formed integrally as a distal portion of the delivery element 410, and an actuating mechanism 430. The delivery element 410, detach mechanism 420 and actuating mechanism 430 may be made of any suitable material such as a polymer or metal. In particular, in the illustrated embodiment the distal portion of the delivery element 410 comprising the detach mechanism 420 is made of a resiliently deformable material (e.g. resiliently deformable polymer or metal). The detach mechanism 420 is configured to be in the first position, configured to grip a medical implant, in a relaxed configuration. The detach mechanism 420 has a first configuration in which the detach mechanism 420 is configured to grip the implant, as shown in Fig. 14A, and a second configuration in which the detach mechanism 420 is configured to release the implant, as shown in Fig. 14B. The actuating mechanism 430 extends through a lumen of the delivery element 410. The delivery element 410 prevents the actuating mechanism 420 from buckling. The actuating mechanism 420 is movable between a first position and a second position. The first position is shown in Fig. 14A.

The actuating mechanism 420 of Fig. 14A comprises a distal part 434. The distal part 434 comprises one or more ramps 442a, 442b received by one or more corresponding ramps 440a, 440b on the distal part of the delivery element 410. When the actuating mechanism 432 is moved distally relative to the delivery element 410, the ramps 442 push the actuating mechanism 420 outwards to a second position for releasing the medical implant, as shown in Fig. 14B. The distal part 434 may comprise a proximal shoulder configured to abut a corresponding shoulder on the delivery element 410, to form a stopper mechanism to prevent displacement of the actuating mechanism 430 beyond a most proximal position. The gripping elements 420a, 420b may be claws comprising inwardly facing teeth in order to assist in gripping the embolization device.

Fig. 15A shows a delivery system for delivering and deploying an implant to a bodily lumen according to one or more embodiments. The delivery system comprises a delivery element 410 comprising a plurality of elongate elements configured to extend through a lumen of a delivery catheter, a detach mechanism 420 formed integrally as a distal portion of the elongate elements of delivery element 410, and an actuating mechanism 430 comprising a sheath 450. The delivery element 410, detach mechanism 420 and actuating mechanism 430 may be made of any suitable material such as a polymer or metal. In particular, in the illustrated embodiment the distal portion of the delivery element 410 comprising the detach mechanism 420 is made of a resiliently deformable material (e.g. resiliently deformable polymer or metal). The detach mechanism 420 is configured to be in the second position, configured to release a medical implant, in a relaxed configuration. The detach mechanism 420 has a first configuration in which the detach mechanism 420 is configured to grip the implant, as shown in Fig. 15A, and a second configuration in which the detach mechanism 420 is configured to release the implant, as shown in Fig. 15B. The actuating mechanism 420 is movable between a first position and a second position. The first position is shown in Fig. 15A. The sheath 450 and delivery element 410 may comprise corresponding stopping elements as disclosed in other embodiments.

In the first position shown in Fig 15A, the sheath 450 prevents the detach mechanism 420 from moving to the relaxed configuration (i.e. the second position for releasing the medical implant). When the sheath 450 is moved proximally relative to the delivery element 410, the distal part of the delivery element 410 is exposed from the distal end of the sheath 450 and the detach mechanism 420 is free to move to the second position for releasing the medical implant, and shown in Fig. 15B.

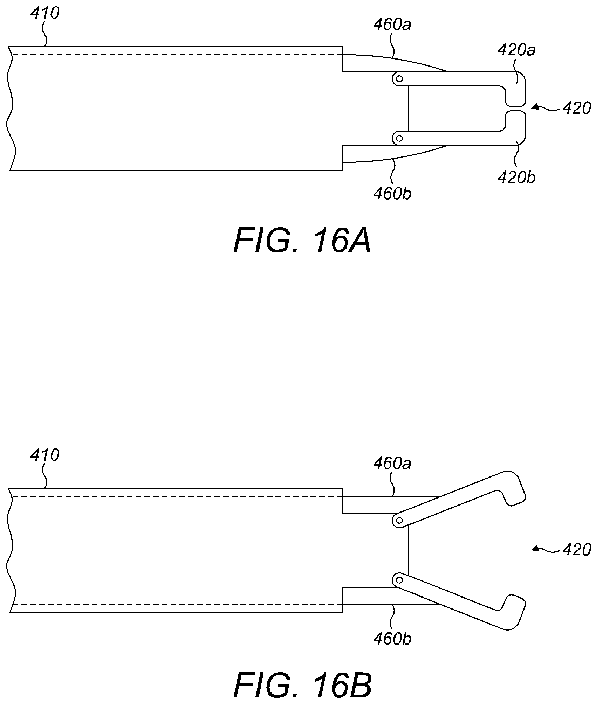

Fig, 16A shows a delivery system for delivering and deploying an implant to a bodily lumen according to one or more embodiments. The delivery system comprises a delivery element 410 configured to extend through a lumen of a delivery catheter, a detach mechanism 420 and an actuating mechanism 430 comprising elongate elements 460a, 460b (e.g. wires or ribbons). The detach mechanism 430 comprises a pair of gripping elements hingedly mounted to a distal part of the delivery element 410 and has a first configuration in which the detach mechanism 430 is configured to grip the implant, as shown in Fig. 16A, and a second configuration in which the detach mechanism is configured to release the implant as shown in Fig. 16B. The elongate elements 460a, 460b may be received inside one or more lumens of the delivery element 410. The gripping elements 420a, 420b may be claws comprising inwardly facing teeth in order to assist in gripping the medical implant.

The elongate elements 460a, 460b are respectively connected to gripping elements 420a, 420b. Translation of the elongate elements 460a, 460b in a proximal direction relative to the delivery element 410 causes the gripping elements to move from the first position to the second position, as shown in Fig. 16B.

The delivery systems described with respect to Figs. 14A-17B can be used to deploy a medical implant in the manner described with respect to Figs. 11A to 11C.

The delivery systems disclosed herein may additionally be provided with a delivery catheter configured to fit the delivery system within a lumen of the delivery catheter.

Fig. 18 shows a schematic view of an embolization device 110, which may have any of the features of the embolization device 110 described with respect to Fig. 1. The embolization device 110 is connected to a flexible joint 130 which is the same as that described with respect to Fig. 3A. The flexible joint 130 may be any of the flexible joints described with respect to Figs. 3A to 9D. A gripping feature 555 is provided proximal to the flexible joint. The gripping feature 555 is configured to be gripped by a delivery system such as any of those described with reference to Figs. 10A to 17B. Accordingly, there may be provided an embolization system comprising an embolization device 110, the embolization device 110 comprising a self-expandable skeleton and a flow restricting layer mounted on the skeleton, the embolization device 100 having a collapsed delivery configuration in which the embolization device is configured to fit inside a delivery catheter, and an expanded deployed configuration in which the skeleton is configured to anchor the embolization device to a bodily lumen; wherein in the expanded deployed configuration the flow restricting layer extends across the bodily lumen to restrict blood flow through the bodily lumen, wherein the embolization further comprises a detach mechanism for connecting the embolization device to a delivery element and actuatable to release the embolization device from the delivery element; a flexible joint 130 having a higher flexibility than the embolization device 110, the flexible joint for allowing the embolization device 110 to tilt with respect to the delivery element when the delivery element is connected to the embolization device; a delivery element configured to extend through a lumen of a delivery catheter; a detach mechanism connected to a distal portion of the delivery element, the detach mechanism having a first configuration in which the detach mechanism is configured to grip the embolization device 110 and a second configuration in which the detach mechanism is configured to release the embolization device 110; and an actuating mechanism configured to extend through the lumen of the delivery catheter to the detach mechanism, the actuating mechanism movable between a first position and a second position, wherein moving the actuating mechanism from the first position to the second position changes the detach mechanism from the first configuration to the second configuration. Accordingly, any of the embolization systems described with reference to Figs. 3A to 9D in which the flexible joint 130 is provided distally to the detach mechanism 140 may comprise a gripping feature proximal to the flexible joint 130 instead of the screw mechanism or electrolytic element and the delivery element 150 may comprise any detach mechanism configured to grip the gripping element as described in relation to Figs. 10A to 17B.

In light of this, there will be many alternatives which implement the teaching of the present disclosure. It is expected that one skilled in the art will be able to modify and adapt the above disclosure to suit its own circumstances and requirements within the scope of the present disclosure, while retaining some or all technical effects of the same, either disclosed or derivable from the above, in light of his common general knowledge in this

## Claims

1. A delivery system (400) for delivering and deploying an implant to a bodily lumen (600), comprising:
a delivery element (410) configured to extend through a lumen of a delivery catheter (500);
a detach mechanism (420) connected to a distal portion of the delivery element (410), the detach mechanism (420) having a first configuration in which the detach mechanism (420) is configured to grip the implant and a second configuration in which the detach mechanism (420) is configured to release the implant; and
an actuating mechanism (430) configured to extend through the lumen of the delivery catheter (500) to the detach mechanism (420), the actuating mechanism (430) movable between a first position and a second position, wherein moving the actuating mechanism (430) from the first position to the second position changes the detach mechanism (420) from the first configuration to the second configuration;
further comprising a first stopping element (417) on the delivery element (410) configured to abut with a second stopping element (419) on the detach mechanism (420) at a predetermined relative position of the detach mechanism (420) and delivery element (410), to prevent movement of the detach mechanism (420) relative to the delivery element (410) beyond a most proximal and/or most distal position.

2. The delivery system (400) of claim 1, wherein the detach mechanism (420) comprises a pair of gripping elements (420a, 420b).

3. The delivery system (400) of claim 2, wherein the gripping elements (420a, 420b) are claws comprising inwardly facing teeth to grip the implant.

4. The delivery system (400) of claim 2 or 3, wherein the gripping elements (420a, 420b) are hingedly connected to the delivery element (410).

5. The delivery system (400) of claim 4, wherein the actuating mechanism (430) comprises an elongate element (432) connected to both of the pair of gripping elements (420a, 420b) for simultaneously actuating both of the pair of gripping elements (420a, 420b).

6. The delivery system (400) of claim 4 or 5, wherein the actuating mechanism (430) is slidably received within one or more lumen (415) of the delivery element (410).

7. The delivery system (400) of any of claims 2 to 4 wherein the gripping elements (420a, 420b) comprise a resiliently deformable material on a distal part of the delivery element (410).

8. The delivery system (400) of claim 7 wherein the gripping elements (420a, 420b) have a relaxed configuration in the first position and the actuating mechanism (430) comprises a distal part (434) comprising one or more ramps (440a, 440b) received by one or more corresponding ramps (440a, 440b) in the delivery element (410), the ramps (440a, 440b) configured to move the gripping elements (420a, 420b) to the second position when the actuating mechanism (430) is moved distally.

9. The delivery system (400) of claim 7, wherein the gripping elements (420a, 420b) have a relaxed configuration in the second position and the actuating mechanism (430) comprises a sheath (450) configured to maintain the gripping elements (420a, 420b) in the first position.

10. An embolization system comprising the delivery system (400) of any preceding claim and an implant, wherein the implant is an embolization device (110), comprising a self-expandable skeleton (112) and a flow restricting layer (114) mounted on the skeleton (112), the embolization device having a collapsed delivery configuration in which the embolization device is configured to fit inside a delivery catheter (500), and an expanded deployed configuration in which the skeleton (112) is configured to anchor the embolization device (110) to a bodily lumen (600); wherein in the expanded deployed configuration the flow restricting layer (114) extends across the bodily lumen (600) to restrict blood flow through the bodily lumen (600);
the embolization device comprising a gripping feature (555) configured to be gripped by the delivery system (400); and
the embolization system (400) comprising a flexible joint (130) distal to the gripping feature (555) and having a higher flexibility than the embolization device (110), the flexible joint (130) for allowing the embolization device (110) to tilt with respect to the delivery element (410) when the delivery element (410) is connected to the embolization device (110).

11. The embolization system of claim 10, wherein the flexible joint (130) comprises two or more connected loops (300a, 300b).

12. The embolization system of claim 10, wherein the flexible joint (130) comprises a hinge pin (302) and a hinge loop (304) connected to the hinge pin (302).

13. The embolization system of claim 10, wherein the flexible joint (130) comprises a resiliently deformable joint, optionally a spring (306).

14. The embolization system of claim 13, further comprising an inextensible element (308) extending across the flexible joint (130).

15. The embolization system of claim 10, wherein the flexible joint (130) comprises a flexible material (310) or a flexible tube (320).

## Patentansprüche

1. Freisetzungssystem (400) zum Freisetzen und Einsetzen eines Implantats in ein Körperlumen (600), umfassend:
ein Freisetzungselement (410), das dazu ausgebildet ist, sich durch ein Lumen eines Freisetzungskatheters (500) zu erstrecken;
einen Ablösemechanismus (420), der mit einem distalen Abschnitt des Freisetzungselements (410) verbunden ist, wobei der Ablösemechanismus (420) eine erste Konfiguration aufweist, in der der Ablösemechanismus (420) dazu ausgebildet ist, das Implantat zu greifen, und eine zweite Konfiguration, in der der Ablösemechanismus (420) dazu ausgebildet ist, das Implantat freizugeben; und
einen Betätigungsmechanismus (430), der dazu ausgebildet ist, sich durch das Lumen des Freisetzungskatheters (500) bis zu dem Ablösemechanismus (420) zu erstrecken, wobei der Betätigungsmechanismus (430) zwischen einer ersten Position und einer zweiten Position beweglich ist, wobei das Bewegen des Betätigungsmechanismus (430) von der ersten Position in die zweite Position, den Ablösemechanismus (420) von der ersten Konfiguration in die zweite Konfiguration überführt;
weiter umfassend ein erstes Anschlagelement (417) an dem Freisetzungselement (410), das so ausgebildet ist, dass es mit einem zweiten Anschlagelement (419) an dem Ablösemechanismus (420) an einer vorbestimmten relativen Position des Ablösemechanismus (420) und des Freisetzungselements (410) anschlägt, um eine Bewegung des Ablösemechanismus (420) relativ zu dem Freisetzungselement (410) über die proximale und/oder distale Endlage hinaus zu verhindern.

2. Freisetzungssystem (400) nach Anspruch 1, wobei der Ablösemechanismus (420) ein Greifelementpaar (420a, 420b) umfasst.

3. Freisetzungssystem (400) nach Anspruch 2, wobei die Greifelemente (420a, 420b) Krallen sind, die nach innen gerichtete Zähne umfassen, um das Implantat zu greifen.

4. Freisetzungssystem (400) nach Anspruch 2 oder 3, wobei die Greifelemente (420a, 420b) gelenkig mit dem Freisetzungselement (410) verbunden sind.

5. Freisetzungssystem (400) nach Anspruch 4, wobei der Betätigungsmechanismus (430) ein längliches Element (432) umfasst, das mit beiden Greifelementen des Greifelementpaars (420a, 420b) verbunden ist, um beide Greifelemente des Greifelementpaars (420a, 420b) gleichzeitig zu betätigen.

6. Freisetzungssystem (400) nach Anspruch 4 oder 5, wobei der Betätigungsmechanismus (430) gleitend in einem oder mehreren Lumen (415) des Freisetzungselements (410) aufgenommen ist.

7. Freisetzungssystem (400) nach einem der Ansprüche 2 bis 4, wobei die Greifelemente (420a, 420b) ein elastisch verformbares Material an einem distalen Teil des Freisetzungselements (410) umfassen.

8. Freisetzungssystem (400) nach Anspruch 7, wobei die Greifelemente (420a, 420b) in der ersten Position eine entspannte Konfiguration aufweisen und der Betätigungsmechanismus (430) einen distalen Teil (434) umfasst, der eine oder mehrere Rampen (440a, 440b) umfasst, die von einer oder mehreren korrespondierenden Rampen (440a, 440b) in dem Freisetzungselement (410) aufgenommen werden, wobei die Rampen (440a, 440b) dazu ausgebildet sind, die Greifelemente (420a, 420b) in die zweite Position zu bewegen, wenn der Betätigungsmechanismus (430) distal bewegt wird.

9. Freisetzungssystem (400) nach Anspruch 7, wobei die Greifelemente (420a, 420b) in der zweiten Position eine entspannte Konfiguration aufweisen und der Betätigungsmechanismus (430) eine Hülse (450) umfasst, die dazu ausgebildet ist, die Greifelemente (420a, 420b) in der ersten Position zu halten.

10. Embolisationssystem, umfassend das Freisetzungssystem (400) nach einem vorstehenden Anspruch und ein Implantat, wobei das Implantat eine Embolisationsvorrichtung (110) ist, umfassend ein selbstexpandierendes Skelett (112) und eine auf dem Skelett (112) angeordnete Durchflussbegrenzungsschicht (114), wobei die Embolisationsvorrichtung eine kontrahierte Freisetzungskonfiguration aufweist, in der die Embolisationsvorrichtung dazu ausgebildet ist, in einen Freisetzungskatheter (500) zu passen, und eine expandierte Einsatzkonfiguration, in der das Skelett (112) dazu ausgebildet ist, die Embolisationsvorrichtung (110) an einem Körperlumen (600) zu verankern; wobei sich in der expandierten Einsatzkonfiguration die Durchflussbegrenzungsschicht (114) über das Körperlumen (600) erstreckt, um den Blutfluss durch das Körperlumen (600) zu begrenzen;
wobei die Embolisationsvorrichtung ein Greifmerkmal (555) umfasst, das dazu ausgebildet ist, von dem Freisetzungssystem (400) gegriffen zu werden; und
wobei das Embolisationssystem (400) ein flexibles Gelenk (130) distal des Greifmerkmals (555) umfasst und eine höhere Flexibilität als die Embolisationsvorrichtung (110) aufweist, wobei das flexible Gelenk (130) zulässt, die Embolisationsvorrichtung (110) relativ zu dem Freisetzungselement (410) zu verschwenken, wenn das Freisetzungselement (410) mit der Embolisationsvorrichtung (110) verbunden ist.

11. Embolisationssystem nach Anspruch 10, wobei das flexible Gelenk (130) zwei oder mehr verbundene Schlaufen (300a, 300b) umfasst.

12. Embolisationssystem nach Anspruch 10, wobei das flexible Gelenk (130) einen Scharnierbolzen (302) und eine Scharnieröse (304) umfasst, die mit dem Scharnierbolzen (302) verbunden ist.

13. Embolisationssystem nach Anspruch 10, wobei das flexible Gelenk (130) ein elastisch verformbares Gelenk umfasst, optional eine Feder (306).

14. Embolisationssystem nach Anspruch 13, wobei das Embolisationssystem weiter ein nicht dehnbares Element (308) umfasst, das sich über das flexible Gelenk (130) erstreckt.

15. Embolisationssystem nach Anspruch 10, wobei das flexible Gelenk (130) ein flexibles Material (310) oder einen flexiblen Schlauch (320) umfasst.

## Revendications

1. Système (400) de pose pour poser et déployer un implant dans une lumière corporelle (600), comprenant :
un élément de pose (410) configuré pour s'étendre à travers une lumière d'un cathéter de pose (500) ;
un mécanisme de détachement (420) relié à une portion distale de l'élément de pose (410), le mécanisme de détachement (420) présentant une première configuration dans laquelle le mécanisme de détachement (420) est configuré pour saisir l'implant et une deuxième configuration dans laquelle le mécanisme de détachement (420) est configuré pour libérer l'implant ; et
un mécanisme d'actionnement (430) configuré pour s'étendre à travers la lumière du cathéter de pose (500) jusqu'au mécanisme de détachement (420), le mécanisme d'actionnement (430) étant mobile entre une première position et une deuxième position, dans lequel le déplacement du mécanisme d'actionnement (430) de la première position vers la deuxième position modifie le mécanisme de détachement (420) de la première configuration à la deuxième configuration ;
comprenant en outre un premier élément de butée (417) sur l'élément de pose (410) configuré pour venir en butée avec un deuxième élément de butée (419) sur le mécanisme de détachement (420) au niveau d'une position relative prédéterminée du mécanisme de détachement (420) et de l'élément de pose (410), pour empêcher un déplacement du mécanisme de détachement (420) par rapport à l'élément de pose (410) au-delà d'une position la plus proximale et/ou la plus distale.

2. Système (400) de pose selon la revendication 1, dans lequel le mécanisme de détachement (420) comprend une paire d'éléments de saisie (420a, 420b).

3. Système (400) de pose selon la revendication 2, dans lequel les éléments de saisie (420a, 420b) sont des griffes comprenant des dents orientées vers l'intérieur pour saisir l'implant.

4. Système (400) de pose selon la revendication 2 ou 3, dans lequel les éléments de saisie (420a, 420b) sont reliés de manière articulée à l'élément de pose (410).

5. Système (400) de pose selon la revendication 4, dans lequel le mécanisme d'actionnement (430) comprend un élément allongé (432) relié aux deux éléments de la paire d'éléments de saisie (420a, 420b) pour actionner simultanément les deux éléments de la paire d'éléments de saisie (420a, 420b).

6. Système (400) de pose selon la revendication 4 ou 5, dans lequel le mécanisme d'actionnement (430) est reçu de manière coulissante à l'intérieur d'une ou plusieurs lumières (415) de l'élément de pose (410).

7. Système (400) de pose selon l'une quelconque des revendications 2 à 4, dans lequel les éléments de saisie (420a, 420b) comprennent un matériau déformable de manière élastique sur une partie distale de l'élément de pose (410).

8. Système (400) de pose selon la revendication 7, dans lequel les éléments de saisie (420a, 420b) présentent une configuration au repos dans la première position et le mécanisme d'actionnement (430) comprend une partie distale (434) comprenant une ou plusieurs rampes (440a, 440b) reçues par une ou plusieurs rampes (440a, 440b) correspondantes dans l'élément de pose (410), les rampes (440a, 440b) étant configurées pour déplacer les éléments de saisie (420a, 420b) vers la deuxième position lorsque le mécanisme d'actionnement (430) est déplacé distalement.

9. Système (400) de pose selon la revendication 7, dans lequel les éléments de saisie (420a, 420b) présentent une configuration au repos dans la deuxième position et le mécanisme d'actionnement (430) comprend une gaine (450) configurée pour maintenir les éléments de saisie (420a, 420b) dans la première position.

10. Système d'embolisation comprenant le système (400) de pose selon une quelconque revendication précédente et un implant, dans lequel l'implant est un dispositif d'embolisation (110), comprenant une armature (112) auto-expansible et une couche de limitation de flux (114) montée sur l'armature (112), le dispositif d'embolisation présentant une configuration de pose affaissée dans laquelle le dispositif d'embolisation est configuré pour s'adapter à l'intérieur d'un cathéter de pose (500), et une configuration déployée expansée dans laquelle l'armature (112) est configurée pour ancrer le dispositif d'embolisation (110) à une lumière corporelle (600) ; dans lequel, dans la configuration déployée expansée, la couche de limitation de flux (114) s'étend à travers la lumière corporelle (600) pour limiter le flux sanguin à travers la lumière corporelle (600) ;
le dispositif d'embolisation comprenant un élément de saisie (555) configuré pour être saisi par le système (400) de pose ; et
le système (400) d'embolisation comprenant un raccord flexible (130) situé en position distale par rapport à l'élément de saisie (555) et présentant une flexibilité supérieure à celle du dispositif d'embolisation (110),
le raccord flexible (130) étant destiné à permettre une inclinaison du dispositif d'embolisation (110) par rapport à l'élément de pose (410) lorsque l'élément de pose (410) est relié au dispositif d'embolisation (110).

11. Système d'embolisation selon la revendication 10, dans lequel le raccord flexible (130) comprend deux ou plus de deux boucles (300a, 300b) reliées.

12. Système d'embolisation selon la revendication 10, dans lequel le raccord flexible (130) comprend une broche de charnière (302) et une boucle de charnière (304) reliée à la broche de charnière (302).

13. Système d'embolisation selon la revendication 10, dans lequel le raccord flexible (130) comprend un raccord déformable de manière élastique, facultativement un ressort (306).

14. Système d'embolisation selon la revendication 13, comprenant en outre un élément inextensible (308) s'étendant à travers le raccord flexible (130).

15. Système d'embolisation selon la revendication 10, dans lequel le raccord flexible (130) comprend un matériau flexible (310) ou un tube flexible (320).
